# NEW EUROPEAN PATENT SPECIFICATION

(11) **EP 1 931 350 B2**
(45) Date of publication and mention of the opposition decision: **04.08.2021**
(45) Mention of the grant of the patent: 20.11.2013
(21) Application number: 06803523.7
(22) Date of filing: 13.09.2006
(51) Int. Cl.: A61K 31/513, A61K 31/44, A61K 31/131, A61P 3/10

(54) **ADMINISTRATION OF DIPEPTIDYL PEPTIDASE INHIBITORS**
VERABREICHUNG VON DIPEPTIDYL-PEPTIDASE-HEMMERN
ADMINISTRATION D'INHIBITEURS DE DIPEPTIDYL PEPTIDASE

(30) Priority: 14.09.2005 US 717558 P; 15.05.2006 US 747273 P
(43) Date of publication of application: 18.06.2008
(73) Proprietor: Takeda Pharmaceutical Company Limited, Osaka-shi, Osaka 541-0045 (JP)
(72) Inventor: CHRISTOPHER, Ronald, J, Carlsbad, California 92011 (US); COVINGTON, Paul, Wilmington, North Carolina 28409 (US)
(74) Representative: D Young & Co LLP
(86) International application number: PCT/US2006/035708
(87) International publication number: WO 2007/033266

(56) References cited:
- EP-A1- 1 586 571
- WO-A-2005/016911
- WO-A-2005/095381
- WO-A1-2005/026148
- WO-A2-2004/018468
- WO-A2-2004/087053
- WO-A2-2005/030751
- 'Safety and Efficacy Study of Alogliptin on Glycemic Control in Subjects with Type 2 Diabetes, March 2005
- WHO Drug Information, Vol. 20, No. 4, 2006, page 282 (post-published evidence regarding the structural formula of Alogliptin)
- Zhang et al: J. Medi. Chemi., vol. 54, 2011, pages 510-524,

## Description

### FIELD OF THE INVENTION

The invention relates to pharmaceutical compositions containing compounds used to inhibit dipeptidyl peptidase IV.

### DESCRIPTION OF RELATED ART

Dipeptidyl Peptidase IV (IUBMB Enzyme Nomenclature EC.3.4.14.5) is a type II membrane protein that has been referred to in the literature by a wide a variety of names including DPP4, DP4, DAP-IV, FAPβ, adenosine deaminase complexing protein 2, adenosine deaminase binding protein (ADAbp), dipeptidyl aminopeptidase IV; Xaa-Pro-dipeptidyl-aminopeptidase; Gly-Pro naphthylamidase; postproline dipeptidyl aminopeptidase IV; lymphocyte antigen CD26; glycoprotein GP110; dipeptidyl peptidase IV; glycylproline aminopeptidase; glycylproline aminopeptidase; X-prolyl dipeptidyl aminopeptidase; pep X; leukocyte antigen CD26; glycylprolyl dipeptidylaminopeptidase; dipeptidyl-peptide hydrolase; glycylprolyl aminopeptidase; dipeptidyl-aminopeptidase IV; DPP IV/CD26; amino acyl-prolyl dipeptidyl aminopeptidase; T cell triggering molecule Tp103; X-PDAP. Dipeptidyl Peptidase IV is referred to herein as "DPP-IV."

DPP-IV is a non-classical serine aminodipeptidase that removes Xaa-Pro dipeptides from the amino terminus (N-terminus) of polypeptides and proteins. DPP-IV dependent slow release of dipeptides of the type X-Gly or X-Ser has also been reported for some naturally occurring peptides.

DPP-IV is constitutively expressed on epithelial and endothelial cells of a variety of different tissues (intestine, liver, lung, kidney and placenta), and is also found in body fluids. DPP-IV is also expressed on circulating T-lymphocytes and has been shown to be synonymous with the cell-surface antigen, CD-26.

DPP-IV is responsible for the metabolic cleavage of certain endogenous peptides (GLP-1 (7-36), glucagon) *in vivo* and has demonstrated proteolytic activity against a variety of other peptides (GHRH, NPY, GLP-2, VIP) *in vitro.*

GLP-1 (7-36) is a 29 amino-acid peptide derived by post-translational processing of proglucagon in the small intestine. GLP-1 (7-36) has multiple actions *in vivo* including the stimulation of insulin secretion, inhibition of glucagon secretion, the promotion of satiety, and the slowing of gastric emptying. Based on its physiological profile, the actions of GLP-1 (7-36) are believed to be beneficial in the prevention and treatment of type II diabetes and potentially obesity. For example, exogenous administration of GLP-1 (7-36) (continuous infusion) in diabetic patients has been found to be efficacious in this patient population. Unfortunately, GLP-1 (7-36) is degraded rapidly *in vivo* and has been shown to have a short half-life *in vivo* (t_{1/2}=1.5 minutes).

Based on a study of genetically bred DPP-IV knock out mice and on *in vivo* / *in vitro* studies with selective DPP-IV inhibitors, DPP-IV has been shown to be the primary degrading enzyme of GLP-1 (7-36) *in vivo.* GLP-1 (7-36) is degraded by DPP-IV efficiently to GLP-1 (9-36), which has been speculated to act as a physiological antagonist to GLP-1 (7-36). Inhibiting DPP-IV *in vivo* is therefore believed to be useful for potentiating endogenous levels of GLP-1 (7-36) and attenuating the formation of its antagonist GLP-1 (9-36). Thus, DPP-IV inhibitors are believed to be useful agents for the prevention, delay of progression, and/or treatment of conditions mediated by DPP-IV, in particular diabetes and more particularly, type 2 diabetes mellitus, diabetic dislipidemia, conditions of impaired glucose tolerance (IGT), conditions of impaired fasting plasma glucose (IFG), metabolic acidosis, ketosis, appetite regulation and obesity.

Several compounds have been shown to inhibit DPP-IV. Nonetheless, a need still exists for new DPP-IV inhibitors and methods of administering such inhibitors for the treatment of disease.

### SUMMARY OF THE INVENTION

Pharmaceutical compositions are provided according to the present invention. The invention is as defined in the appended claims.
In one embodiment, a pharmaceutical composition is provided that is formulated in a single dose form wherein such single dose form comprises between 5 mg and 250 mg of Compound I, wherein Compound I is present in the pharmaceutical composition in a benzoate salt and has the formula 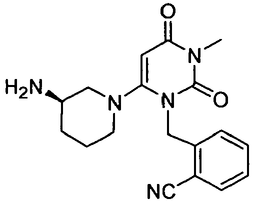 with the exception of the following compositions: (a) a pharmaceutical composition formulated for oral administration consisting of 10 to 100 mg of Compound I, 105 mg of citric acid monohydrate, 18 mg of sodium hydroxide, a flavouring and water to 100 ml; and (b) a pharmaceutical composition formulated for intravenous administration consisting of 5 to 10 mg of Compound I, 1.05 mg of citric acid monohydrate, 0.18 mg of sodium hydroxide, a sufficient quantity of dextrose monohydrate to make the formulation isotonic, and water to 1 ml. In another aspect there is provided a pharmaceutical composition formulated in a single dose form wherein such single dose form comprises 12.5 mg, 20 mg, 25mg, 50 mg or 75 mg of Compound I. The structure of compound I is described below.

In another embodiment, a pharmaceutical composition is provided that comprises Compound I and one or more antidiabetic compounds other than Compound I in a single dose form. Compound I is present in the single dose form in a dosage amount between 5 and 250 mg of Compound I, optionally between 10 mg and 200 mg of Compound I, optionally between 10 mg and 150 mg of Compound I, and optionally between 10 mg and 100 mg of Compound I. In particular variations, the pharmaceutical composition comprises 10 mg, 12.5 mg, 20 mg, 25mg, 50 mg, 75 mg or 100 mg of Compound I.

Combination of Compound I with one or more antidiabetic compounds other than Compound I provides excellent effects such as 1) enhancement in therapeutic effects of Compound I and/or the antidiabetic compounds; 2) reduction in side effects of Compound I and/or the antidiabetic compounds; and 3) reduction in a dose of Compound I and/or the antidiabetic compounds.

According to above embodiment, the one or more antidiabetic compounds comprised in the pharmaceutical composition may optionally be selected from the group consisting of insulin signaling pathway modulators, compounds influencing a dysregulated hepatic glucose production, insulin sensitivity enhancers, and insulin secretion enhancers. Also according to above embodiment, the one or more antidiabetic compounds comprised in the pharmaceutical composition may optionally be selected from the group consisting of protein tyrosine phosphatase inhibitors, glutamine-fructose-6-phosphate amidotransferase inhibitors, glucose-6-phosphatase inhibitors, fructose-1,6-bisphosphatase inhibitors, glycogen phosphorylase inhibitors, glucagon receptor antagonists, phosphoenolpyruvate carboxykinase inhibitors, pyruvate dehydrogenase kinase inhibitors, alpha-glucosidase inhibitors, inhibitors of gastric emptying, glucokinase activators, GLP-1 receptor agonists, GLP-2 receptor agonists, UCP modulators, RXR modulators, GSK-3 inhibitors, PPAR modulators, metformin, insulin, and α₂-adrenergic antagonists.

Also according to above embodiment, the one or more antidiabetic compounds comprised in the pharmaceutical composition may optionally be selected from the group consisting of GSK-3 inhibitors, retinoid X receptor agonists, Beta-3 AR agonists, UCP modulators, antidiabetic thiazolidinediones, non-glitazone type PPAR gamma agonists, dual PPAR gamma/PPAR alpha agonists, antidiabetic vanadium containing compounds and biguanides.

Also according to above embodiment, the one or more antidiabetic compounds comprised in the pharmaceutical composition may optionally be thiazolidinediones selected from the group consisting of (S)-((3,4-dihydro-2-(phenyl-methyl)-2H-1-benzopyran-6-yl)methyl-thiazolidine-2,4-dione, 5-{[4-(3-(5-methyl-2-phenyl-4-oxazolyl)-l-oxo-propyl)-phenyl]-methyl}-thiazolidine-2,4-dione, 5-{[4-(1-methylcyclohexyl)methoxy)-phenyl]methyl]-thiazolidine-2,4-dione, 5-{[4-(2-(1-indolyl)ethoxy)phenyl]methyl}-thiazolidine-2,4-dione, 5-{4-[2-(5-methyl-2-phenyl-4-oxazoly)-ethoxy)]benzyl}-thiazolidine-2,4-dione, 5-(2-naphthylsulfonyl)-thiazolidine-2,4-dione, bis{4-[(2,4-dioxo-5-thiazolidinyl)-methyl]phenyl}methane, 5-{4-[2-(5-methyl-2-phenyl-4-oxazolyl)-2-hydroxyethoxy]-benzyl}- -thiazolidine-2,4-dione, 5-[4-(1-phenyl-1-cyclopropanecarbonylamino)-benzyl]-thiazolidine-2,4-dione, 5-{[4-(2-(2,3-dihydroindol-1-yl)ethoxy)phenylmethyl)-thiazolidine-2,4-dione, 5-[3-(4-chloro-phenyl])-2--propynyl]-5-phenylsulfonyl)thiazolidine-2,4-dione, 5-[3-(4-chlorophenyl])--2-propynyl]-5-(4-fluorophenyl-sulfonyl)thiazolidine-2,4-dione,5-{[4-(2-(methyl-2-pyridinyl-amino)-ethoxy)phenyl]methyl}-thiazolidine-2,4-dione, 5-{[4-(2-(5-ethyl-2-pyridyl)ethoxy)phenyl]-methyl}-thiazolidine-2,4-dione, 5-{[4-((3,4-dihydro-6-hydroxy-2,5,7,8-tetramethyl-2H-1-benzopyran-2-yl)methoxy)-phenyl]-methyl)-thiazolidine-2,4-dione, 5-[6-(2-fluoro-benzyloxy)-naphthalen-2-ylmethyl]-thiazolidine-2,4-dione, 5-([2-(2-naphthyl)-benzoxazol-5-yl]-methyl}thiazolidine-2,4-dione and 5-(2,4-dioxothiazolidin-5-ylmethyl)-2-methoxy-N-(4-trifluoromethyl-benzyl)benzamide, including any pharmaceutically acceptable salts thereof.

In one variation, the one or more antidiabetic compounds comprised in the pharmaceutical composition includes metformin. In one particular variation, the metformin in this combination comprises one or more pharmaceutically acceptable salts thereof. In another particular variation, the metformin in this combination comprises a metformin HCl salt. In still another particular variation, the metformin in this combination is administered in a daily dose of between 125 and 2550 mg. In yet another variation, the metformin in this combination is administered in a daily dose of between 250 and 2550 mg.

In another variation, the one or more antidiabetic compounds comprised in the pharmaceutical composition includes one or more sulphonyl urea derivatives.

In another variation, the one or more antidiabetic compounds comprised in the pharmaceutical composition includes an antidiabetic compound selected from the group consisting of glisoxepid, glyburide, glibenclamide, acetohexamide, chloropropamide, glibornuride, tolbutamide, tolazamide, glipizide, carbutamide, gliquidone, glyhexamide, phenbutamide, tolcyclamide, glimepiride and gliclazide, including any pharmaceutically acceptable salts thereof. In one variation, the one or more antidiabetic compounds comprised in the pharmaceutical composition includes glimepiride.

In another variation, the one or more antidiabetic compounds comprised in the pharmaceutical composition includes an antidiabetic compound selected from the group consisting of incretin hormones or mimics thereof, beta-cell imidazoline receptor antagonists, and short-acting insulin secretagogues.

In another variation, the one or more antidiabetic compounds comprised in the pharmaceutical composition includes insulin.

In another variation, the one or more antidiabetic compounds comprised in the pharmaceutical composition includes one or more GLP-1 agonists.

In another variation, the one or more antidiabetic compounds comprised in the pharmaceutical composition includes one or more antidiabetic D-phenylalanine derivatives.

In another variation, the one or more antidiabetic compounds comprised in the pharmaceutical composition includes an antidiabetic compound selected from the group consisting of repaglinide, mitiglinide, and nateglinide, including any pharmaceutically acceptable salts thereof. In one variation, the one or more antidiabetic compounds comprised in the pharmaceutical composition includes mitiglinide calcium salt hydrate.

In another variation, the one or more antidiabetic compounds comprised in the pharmaceutical composition includes one or more alpha-Glucosidase inhibitors.

In another variation, the one or more antidiabetic compounds comprised in the pharmaceutical composition includes an antidiabetic compound selected from the group consisting of acarbose, voglibose and miglitol, including any pharmaceutically acceptable salts thereof. In one variation, the one or more antidiabetic compounds comprised in the pharmaceutical composition includes voglibose. In another variation, the voglibose in this combination is administered in a daily dose of between 0.1 and 1 mg.

In another variation, the one or more antidiabetic compounds comprised in the pharmaceutical composition includes rosiglitazone, including any pharmaceutically acceptable salts thereof. In one variation, the rosiglitazone in this combination comprises a rosiglitazone maleate salt.

The one or more antidiabetic compounds comprised in the pharmaceutical composition may also optionally be tesaglitazar, muraglitazar or naveglitazar, including any pharmaceutically acceptable salts thereof.

In another variation, the one or more antidiabetic compounds comprised in the pharmaceutical composition includes pioglitazone, including any pharmaceutically acceptable salts thereof. In one particular variation, the pioglitazone in this combination comprises a pioglitazone HCl salt. In another particular variation, the pioglitazone in this combination is administered in a daily dose of between 7.5 and 60 mg. In still another particular variation, the pioglitazone in this combination is administered in a daily dose of between 15 and 45 mg.

In another variation, the one or more antidiabetic compounds comprised in the pharmaceutical composition includes metformin and pioglitazone. In one particular variation, the pioglitazone in this combination comprises one or more pharmaceutically acceptable salts thereof. In another particular variation, the pioglitazone in this combination comprises a pioglitazone HCl salt. In still another particular variation, the pioglitazone in this combination is administered in a daily dose of between 7.5 and 60 mg. In yet another particular variation, the pioglitazone in this combination is administered in a daily dose of between 15 and 45 mg. In a further variation of each of the above variations, the metformin in this combination comprises one or more pharmaceutically acceptable salts thereof. In still a further variation, the metformin in this combination comprises a metformin HCl salt. In yet a further variation, the metformin in this combination is administered in a daily dose of between 125 and 2550 mg. In still a further variation, the metformin in this combination is administered in a daily dose of between 250 and 2550 mg.

In regard to each of the above embodiments and variations thereof regarding pharmaceutical compositions, Compound I may be administered as a free base or as a pharmaceutically acceptable salt thereof. In particular variations, Compound I is administered as a benzoate salt or a toluenesulfonate salt or a hydrochloric acid salt of Compound I.

Also in regard to each of the above embodiments and variations thereof regarding pharmaceutical compositions, the pharmaceutical composition may optionally be a single dose form adapted for oral administration, optionally a solid formulation adapted for oral administration, and optionally a tablet or capsule adapted for oral administration. The pharmaceutical formulation may also be an extended release formulation adapted for oral administration.

Also in regard to each of the above embodiments and variations thereof regarding pharmaceutical compositions, the pharmaceutical composition may optionally be employed to prevent or treat conditions mediated by DPP-IV such as diabetes and more particularly, type 2 diabetes mellitus; diabetic dislipidemia; impaired glucose tolerance (IGT); impaired fasting plasma glucose (IFG); metabolic acidosis; ketosis; appetite regulation; obesity; complications associated with diabetes including diabetic neuropathy, diabetic retinopathy and kidney disease; hyperlipidemia including hypertriglyceridemia, hypercholesteremia, hypoHDLemia and postprandial hyperlipidemia; arteriosclerosis; hypertension; myocardial infarction, angina pectoris, cerebral infarction, cerebral apoplexy and metabolic syndrome.

Also provided are kits comprising multiple doses of pharmaceutical composition according to the present invention.

In one variation, the kits further comprise instructions which comprise one or more forms of information selected from the group consisting of indicating a disease state for which the pharmaceutical composition is to be administered, storage information for the pharmaceutical composition, dosing information and instructions regarding how to administer the pharmaceutical composition.

Also provided are articles of manufacture comprising multiple doses of pharmaceutical composition according to the present invention. In one variation, the articles of manufacture further comprise packaging materials such as a container for housing the multiple doses of the pharmaceutical composition and or a label indicating one or more members of the group consisting of a disease state for which the compound is to be administered, storage information, dosing information and/or instructions regarding how to administer the composition.

It is noted in regard to all of the above embodiments that the embodiments should be interpreted as being open ended in the sense that the methods may comprise further actions beyond those specified including the administration of other pharmaceutically active materials to a patient. Similarly, unless otherwise specified, the pharmaceutical compositions, kits and articles of manufacture may further include other materials including other pharmaceutically active materials.

### BRIEF DESCRIPTION OF THE FIGURES

Figure 1 provides a table summarizing the primary efficacy endpoint measured following breakfast in the double-blind, placebo-controlled, repeat-dose, multicenter study described in Example 3.
Figure 2 provides a table summarizing HbA_{1c} results by treatment and by time point in the double-blind, placebo-controlled, repeat-dose, multicenter study described in Example 3.
Figure 3 provides a table summarizing fasting fructosamine results by treatment and by time point in the double-blind, placebo-controlled, repeat-dose, multicenter study described in Example 3.
Figure 4 illustrates the observed effect that administering Compound I has on a patient's plasma DPPIV activity.

### DEFINITIONS

Unless otherwise stated, the following terms used in the specification and claims shall have the following meanings for the purposes of this Application.

"Disease" specifically includes any unhealthy condition of an animal or part thereof and includes an unhealthy condition that may be caused by, or incident to, medical or veterinary therapy applied to that animal, i.e., the "side effects" of such therapy.

"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

"Pharmaceutically acceptable salts" means salts which are pharmaceutically acceptable, as defined above, and which possess the desired pharmacological activity. Such salts include, but are not limited to, acid addition salts formed with inorganic acids such as hydrochloric acid, hydrobromic acid, sulfuric acid, nitric acid, phosphoric acid, and the like; or with organic acids such as acetic acid, trifluoroacetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, *o*-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, 2-naphthalenesulfonic acid, *p*-toluenesulfonic acid, camphorsulfonic acid, 4-methylbicyclo[2.2.2]oct-2-ene-1-carboxylic acid, glucoheptonic acid, 4,4'-methylenebis(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid and the like.

Pharmaceutically acceptable salts also include, but are not limited to, base addition salts which may be formed when acidic protons present are capable of reacting with inorganic or organic bases. Acceptable inorganic bases include, but are not limited to, sodium hydroxide, sodium carbonate, potassium hydroxide, aluminum hydroxide and calcium hydroxide. Acceptable organic bases include, but are not limited to, ethanolamine, diethanolamine, triethanolamine, tromethamine, *N*-methylglucamine and the like.

"Therapeutically effective amount" means that amount of a compound which, when administered to an animal for treating a disease, is sufficient to effect such treatment for the disease.

"Treatment" or "treating" means any administration of a therapeutically effective amount of a compound and includes:
(1) preventing the disease from occurring in an animal which may be predisposed to the disease but does not yet experience or display the pathology or symptomatology of the disease,
(2) inhibiting the disease in an animal that is experiencing or displaying the pathology or symptomatology of the disease (i.e., arresting further development of the pathology and/or symptomatology), or
(3) ameliorating the disease in an animal that is experiencing or displaying the pathology or symptomatology of the disease (i.e., reversing the pathology and/or symptomatology).

### DETAILED DESCRIPTION OF THE INVENTION

### 1. 2-[[6-[(3R)-3-AMINO-1-PIPERIDINYL]-3,4-DIHYDRO-3-METHYL-2,4-DIOXO-1(2H)-PYRIMIDINYL]METHYL]-BENZONITRILE AND COMPOSITIONS THEREOF

The present invention relates generally to the administration of 2-[[6-[(3R)-3-amino-1-piperidinyl]-3,4-dihydro-3-methyl-2,4-dioxo-1(2H)-pyrimidinyl]methyl]-benzonitrile (referred to herein as "Compound I") whose structure is provided below.

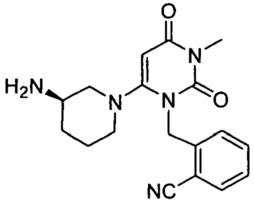

Example 1 describes one method for synthesizing Compound I. It is noted that other methods for synthesizing Compound I may be used as would be appreciated to one of ordinary skill in the art.

Compound I may be administered in its free base form and may also be administered in the form of salts, hydrates and prodrugs that are converted *in vivo* into the free base form of Compound I. For example, it is within the scope of the present invention to administer Compound I as a pharmaceutically acceptable salt derived from various organic and inorganic acids and bases in accordance with procedures well known in the art. As used herein, Compound I is intended to encompass salts, hydrates and prodrugs of Compound I unless otherwise specified.

A pharmaceutically acceptable salt of Compound I preferably confers improved pharmacokinetic properties as compared to the free base form Compound I. Pharmaceutically acceptable salts may also initially confer desirable pharmacokinetic properties on Compound I that it did not previously possess, and may even positively affect the pharmacodynamics of the compound with respect to its therapeutic activity in the body.

Particular examples of salts, hydrates and prodrugs of Compound I include, but are not limited to salt forms formed by inorganic or organic acids, e.g., hydrohalides such as hydrochloride, hydrobromide, hydroiodide; other mineral acids and their corresponding salts such as sulfate, nitrate, phosphate, etc.; alkyl and monoarylsulfonates such as ethanesulfonate, toluenesulfonate and benzenesulfonate; and other organic acids and their corresponding salts such as acetate, trifluoroacetate, tartrate, maleate, succinate, citrate, benzoate, salicylate and ascorbate. Further acid addition salts include, but are not limited to: adipate, alginate, arginate, aspartate, bisulfate, bisulfite, bromide, butyrate, camphorate, camphorsulfonate, caprylate, chloride, chlorobenzoate, cyclopentanepropionate, digluconate, dihydrogenphosphate, dinitrobenzoate, dodecylsulfate, fumarate, galacterate (from mucic acid), galacturonate, glucoheptaoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate and phthalate.

In one variation, Compound I is administered as the benzoate, toluenesulfonate or hydrochloride salt form of Compound I. Example 1 describes the preparation of the benzoate, toluenesulfonate and hydrochloride salt forms of Compound I.

### 2. ADMINISTRATION AND USE OF COMPOUND I

A method comprising administering Compound I to a patient at a daily dose of between 5 mg/day and 250 mg/day of Compound I to a patient, optionally between 10 mg and 200 mg of Compound I, optionally between 10 mg and 150 mg of Compound I, and optionally between 10 mg and 100 mg of Compound I (in each instance based on the molecular weight of the free base form of Compound I), is described herein. Specific dosage amounts that may be used include, but are not limited to 10 mg, 12.5 mg, 20 mg, 25 mg, 50 mg, 75 mg and 100 mg of Compound I per day. It is noted that unless otherwise specifically specified, Compound I may be administered in its free base form or as a pharmaceutically acceptable salt. However, the dosage amounts and ranges provided herein are always based on the molecular weight of the free base form of Compound I.

Compound I may be administered by any route of administration. In particular embodiments, however, the method of the present invention is practiced by administering Compound I orally. This type of administration is advantageous in that it is easy and may be self-administered by the patient.

Compound I may be administered one or more times per day. An advantage of the present invention, however, is that Compound I can be effectively administered at the dosage levels specified herein one time per day and may also be administered as a single dosage form one time a day. By being able to administer Compound I at the dosage levels specified herein only one time per day and orally, it is easier for patients to self-administer Compound I, thus improving the compliance of usage among patients requiring in vivo inhibition of DPP-IV activity.

Advantageously, Compound I is suitable for prolonged continuous use and may be administered to patients for an extended period of time. Accordingly, the method may be performed where Compound I is administered to a patient each day (optionally 1 time daily) for a period of at least 1 month, optionally for at least 3 months, and, if necessary, optionally for the duration of the patients disease profile. Because of the long acting DPP-IV inhibitory affects of Compound I, it is envisioned that a dosing regiment less frequent than once per day may be employed.

Advantageously, Compound I may be administered at any time during the day. Optionally, Compound I is administered daily one time a day where administration occurs in the morning before meals. Because Compound I can stimulate insulin secretion when blood glucose level reaches levels above 100 mg/dl, it may be beneficial to have Compound I in systemic circulation before an elevation in blood glucose levels occurs postprandially.

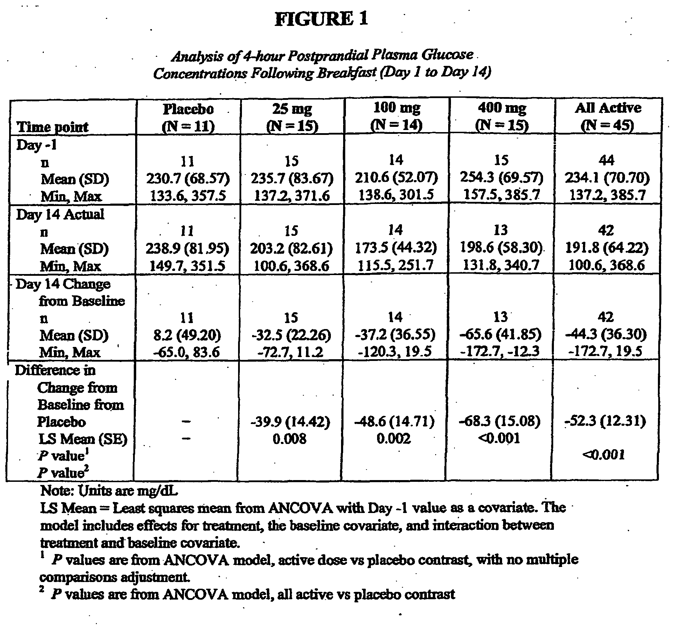

Compound I may be administered to any patient who would benefit from a course of treatment leading to the reduction of in vivo DPP-IV activity. Figure 1 illustrates and Example 3 describes the observed effect that administering Compound I has on a patient's plasma DPPIV activity after 14 days at dosage levels of 25mg/day, 100mg/day and 400 mg/day.

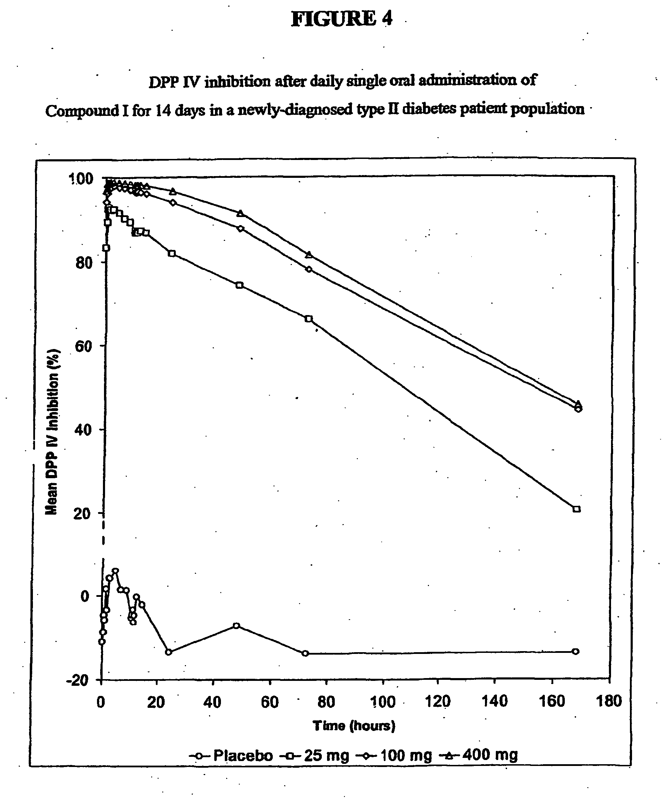

As can be seen from the data shown in Figure 4, by administering Compound I one time per day at the dosage levels specified herein, Compound I can be effectively used relative to disease states where it is desired to reduce the patient's plasma DPPIV activity by greater than 60%, optionally greater than 70%, and optionally greater than 80%. Specifically, when at least 25mg of Compound I is administered, the patient's plasma DPPIV activity may be reduced by greater than 60% relative to baseline for a period of at least at least 6 hours, 12 hours, 18 hours and even 24 hours following administration.

Examples of particular applications for administering Compound I include, but are not limited to the prevention, delay of progression, and/or treatment of conditions mediated by DPP-IV, in particular diabetes and more particularly, type 2 diabetes mellitus, diabetic dislipidemia, impaired glucose tolerance (IGT), impaired fasting plasma glucose (IFG), metabolic acidosis, ketosis, appetite regulation, obesity and complications associated with diabetes including diabetic neuropathy, diabetic retinopathy, inflammatory bowel disease, Crohn's disease, chemotherapy-induced enteritis, oral mucositis, Shorthened Bowel Syndrome and kidney disease. The conditions mediated by DPP-IV further includes hyperlipidemia such as hypertriglyceridemia, hypercholesteremia, hypoHDLemia and postprandial hyperlipidemia; arteriosclerosis; hypertension; myocardial infarction, angina pectoris, cerebral infarction, cerebral apoplexy and metabolic syndrome.

It is believed that administration of Compound I to type I or type II diabetic patients following a minimum treatment of at least 30 days will improve one or more cardiovascular measurements. Examples of cardiac measurements that may be improved include, but are not limited to a decrease in mean systolic blood pressure, an increase in HDL cholesterol, improvement in LDL/HDL ratio and a reduction in triglycerides.

It is also believed that administration of Compound I in combination with one or more antidiabetic compounds to type I or type II diabetic patients following a minimum treatment of at least 30 days will improve one or more cardiovascular measurements. Examples of cardiac measurements that may be improved include, but are not limited to a decrease in mean systolic blood pressure, an increase in HDL cholesterol, improvement in LDL/HDL ratio and a reduction in triglycerides.

In one variation, Compound I is administered to a patient with type 2 diabetes. Patients receiving Compound I may also have a malfunction in insulin secretion from pancreatic islets rather than patients who have developed insulin resistance in peripheral insulin sensitive tissues/organs.

Advantageously, administering Compound I one time per day at the dosage levels specified herein may also be used to treat patients who are prediabetic. It is believed that administering Compound I in a patient who is prediabetic serves to delay development of type II diabetes in that patient. Sustained increase in blood glucose desensitizes pancreatic islet function and impairs insulin secretion. By improving cyclic AMP levels and the calcium dynamics in beta cells, the cells activate genes repairing damaged cell components and are less vulnerable to glucose toxicity.

Administering Compound I one time per day at the dosage levels specified herein is expected to have a range of desirous biological effects *in vivo.* For example, administering Compound I one time per day at the dosage levels specified herein reduces the patient's blood glucose level when compared with placebo control. Such a decrease in postprandial blood glucose levels helps diabetic patients to maintain lower glucose levels.

Administering Compound I one time per day at the dosage levels specified herein is also expected to have the affect of increasing the patient's insulin level or insulin sensitivity. Insulin facilitates entry of glucose into muscle, adipose and several other tissues. The mechanism by which cells can take up glucose is by facilitated diffusion through stimulation of insulin receptor. C-peptide and insulin are protein chains created by the activation and division of proinsulin (an inactive precursor to insulin). C-peptide and insulin are created and stored in the beta cells of the pancreas. When insulin is released into the bloodstream, equal amounts of C-peptide also are released. This makes C-peptide useful as a marker of insulin production. Administering Compound I according to the present invention is expected to increase the patient's C-peptide level.

Administering Compound I one time per day at the dosage levels specified herein is also expected to have the affect of decreasing the patient's hemoglobin A1c level by greater than 0.5% when compared to placebo control after extended treatment with Compound I. Hb-A1c values are known to be directly proportional to the concentration of glucose in the blood over the life span of the red blood cells. Hb-A1c thus gives an indication of a patient's blood glucose levels over the previous last 90 days, skewed to the most recent 30 days. The observed reduction in the patient's hemoglobin A1c level thus verifies the sustained reduction in the patient's blood glucose levels as a result of administering Compound I one time per day at the dosage levels specified herein.

### 3. COMBINATION THERAPY INCLUDING COMPOUND I

The present invention also relates to the use of Compound I formulated in a single dose form wherein such single dose form comprises between 5mg and 250mg of Compound I in combination with one or more antidiabetic compounds other than Compound I. Examples of such other antidiabetic compounds include, but are not limited to insulin signaling pathway modulators, like protein tyrosine phosphatase (PTPase) inhibitors, and glutamine-fructose-6-phosphate amidotransferase (GFAT) inhibitors; compounds influencing a dysregulated hepatic glucose production, like glucose-6-phosphatase (G6Pase) inhibitors, fructose-1,6-bisphosphatase (F-1,6-BPase) inhibitors, glycogen phosphorylase (GP) inhibitors, glucagon receptor antagonists and phosphoenolpyruvate carboxykinase (PEPCK) inhibitors; pyruvate dehydrogenase kinase (PDHK) inhibitors; insulin sensitivity enhancers (insulin sensitizers); insulin secretion enhancers (insulin secretagogues); alpha-glucosidase inhibitors; inhibitors of gastric emptying; glucokinase activators, GLP-1 receptor agonists, UCP modulators, RXR modulators, GSK-3 inhibitors, PPAR modulators, metformin, insulin; and α₂-adrenergic antagonists. Compound I may be administered with such at least one other antidiabetic compound either simultaneously as a single dose, at the same time as separate doses, or sequentially (i.e., where one is administered before or after the other is administered).

Examples of PTPase inhibitors that may be used in combination with Compound I include, but are not limited to those disclosed in U.S. Patent. Nos. 6,057,316, 6,001,867, and PCT Publication Nos. WO 99/58518, WO 99/58522, WO 99/46268, WO 99/46267, WO 99/46244, WO 99/46237, WO 99/46236, and WO 99/15529.

Examples of GFAT inhibitors that may be used in combination with Compound I include, but are not limited to those disclosed in Mol. Cell. Endocrinol. 1997, 135(1), 67-77.

Examples of G6Pase inhibitors that may be used in combination with Compound I include, but are not limited to those disclosed in PCT Publication Nos. WO 00/14090, WO 99/40062 and WO 98/40385, European Patent Publication No. EP682024 and Diabetes 1998, 47,1630-1636.

Examples of F-1,6-BPase inhibitors that may be used in combination with Compound I include, but are not limited to those disclosed in PCT Publication Nos. WO 00/14095, WO 99/47549, WO 98/39344, WO 98/39343 and WO 98/39342.

Examples of GP inhibitors that may be used in combination with Compound I include, but are not limited to those disclosed in U.S. Patent No. 5,998,463, PCT Publication Nos. WO 99/26659, WO 97/31901, WO 96/39384 and WO9639385 and European Patent Publication Nos. EP 978279 and EP 846464.

Examples of glucagon receptor antagonists that may be used in combination with Compound I include, but are not limited to those disclosed in U.S. Patent Nos. 5,880,139 and 5,776,954, PCT Publication Nos. WO 99/01423, WO 98/22109, WO 98/22108, WO 98/21957, WO 97/16442 and WO 98/04528 and those described in Bioorg Med. Chem. Lett 1992, 2, 915-918, J. Med. Chem. 1998, 41, 5150-5157, and J. Biol Chem. 1999, 274; 8694-8697.

Examples of PEPCK inhibitors that may be used in combination with Compound I include, but are not limited to those disclosed in U.S. Patent No. 6,030,837 and Mol. Biol. Diabetes 1994,2, 283-99.

Examples of PDHK inhibitors that may be used in combination with Compound I include, but are not limited to those disclosed in J. Med. Chem. 42 (1999) 2741-2746.

Examples of insulin sensitivity enhancers that may be used in combination with Compound I include, but are not limited to GSK-3 inhibitors, retinoid X receptor (RXR) agonists, Beta-3 AR agonists, UCP modulators, antidiabetic thiazolidinediones (glitazones), non-glitazone type PPAR gamma agonists, dual PPAR gamma/PPAR alpha agonists, antidiabetic vanadium containing compounds and biguanides such as metformin.

Examples of GSK-3 inhibitors include, but are not limited to those disclosed in PCT Publication Nos. WO 00/21927 and WO 97/41854.

Examples of RXR modulators include, but are not limited to those disclosed in U.S. Patent Nos. 4,981,784, 5,071,773, 5,298,429 and 5,506,102 and PCT Publication Nos. WO89/05355, WO91/06677, WO92/05447, WO93/11235, WO95/18380, WO94/23068, and WO93/23431.

Examples of Beta-3 AR agonists include, but are not limited to CL-316,243 (Lederle Laboratories) and those disclosed in U.S. Patent No. 5,705,515 and PCT Publication Nos. WO 99/29672, WO 98/32753, WO 98/20005, WO 98/09625, WO 97/46556, and WO 97/37646.

Examples of UCP modulators include agonists of UCP-1, UCP-2 and UCP-3. Examples of UCP modulators include, but are not limited to those disclosed in Vidal-Puig et al., Biochem. Biophys. Res. Commun., Vol. 235(1) pp. 79-82 (1997).

Examples of antidiabetic, PPAR modulating thiazolidinediones (glitazones) include, but are not limited to, (S)-((3,4-dihydro-2-(phenyl-methyl)-2H-1-benzopyran-6-yl)methyl-thiazolidine-2,4-dione (englitazone), 5-{[4-(3-(5-methyl-2-phenyl-4-oxazolyl)-1-oxo-propyl)-phenyl]-methyl}-thiazolidine-2,4-dione (darglitazone), 5-{[4-(1-methyl-cyclohexyl)methoxy)-phenyl]methyl]-thiazolidine-2,4-dione (ciglitazone), 5-{[4-(2-(1-indolyl)ethoxy)phenyl]methyl}-thiazolidine-2,4-dione (DRF2189), 5-{4-[2-(5-methyl-2-phenyl-4-oxazoly)-ethoxy)]benzyl}-thiazolidine-2,4-dione (BM-13.1246), 5-(2-naphthylsulfonyl)-thiazolidine-2,4-dione (AY-31637), bis{4-[(2,4-dioxo-5-thiazolidinyl)-methyl]phenyl}methane (YM268), 5-{4-[2-(5-methyl-2-phenyl-4-oxazolyl)-2-hydroxyethoxy]-benzyl}- -thiazolidine-2,4-dione (AD-5075), 5-[4-(1-phenyl-1-cyclopropanecarbonylamino)-benzyl]-thiazolidine-2,4-dione (DN-108) 5-{[4-(2-(2,3-dihydroindol-1-yl)ethoxy)phenylmethyl)-thiazolidine-2,4-dione, 5-[3-(4-chloro-phenyl])-2--propynyl]-5-phenylsulfonyl)thiazolidine-2,4-dione, 5-[3-(4-chlorophenyl])--2-propynyl]-5-(4-fluorophenyl-sulfonyl)thiazolidine-2,4-dione,5-{[4-(2-(methyl-2-pyridinyl-amino)-ethoxy)phenyl]methyl}-thiazolidine-2,4-dione (rosiglitazone), 5-{[4-(2-(5-ethyl-2-pyridyl)ethoxy)phenyl]-methyl}-thiazolidine-2,4-dione (pioglitazone; marketed under the trademark ACTOS™), 5-[6-(2-fluoro-benzyloxy)-naphthalen-2-ylmethyl]-thiazolidine-2,4-dione(MCC555), 5-([2-(2-naphthyl)-benzoxazol-5-yl]-methyl}thiazolidine-2,4-dione (T-174), edaglitazone (BM-13-1258), rivoglitazone (CS-011), and 5-(2,4-dioxothiazolidin-5-ylmethyl)-2-methoxy-N-(4-trifluoromethylbenzyl)benzamide (KRP297).

Examples of non-glitazone type PPAR gamma agonists include, but are not limited to N-(2-benzoylphenyl)-L-tyrosine analogues, such as GI-262570, reglixane (JTT501), and FK-614 and metaglidasen (MBX-102).

Examples of dual PPAR gamma/PPAR alpha agonists include, but are not limited to omega.-[(oxoquinazolinylalkoxy)phenyl]alkanoates and analogs thereof including those described in PCT Publication No. WO 99/08501 and Diabetes 2000, 49(5), 759-767; tesaglitazar, muraglitazar and naveglitazar.

Examples of antidiabetic vanadium containing compounds include, but are not limited to those disclosed in the U.S. Patent No. 5,866,563.

Metformin (dimethyldiguanide) and its hydrochloride salt is marketed under the trademark GLUCOPHAGE™.

Examples of insulin secretion enhancers include but are not limited to glucagon receptor antagonists (as described above), sulphonyl urea derivatives, incretin hormones or mimics thereof, especially glucagon-like peptide-1 (GLP-1) or GLP-1 agonists, beta-cell imidazoline receptor antagonists, and short-acting insulin secretagogues, like antidiabetic phenylacetic acid derivatives, antidiabetic D-phenylalanine derivatives, and mitiglinide and pharmaceutical acceptable salts thereof.

Examples of sulphonyl urea derivatives include, but are not limited to, glisoxepid, glyburide, glibenclamide, acetohexamide, chloropropamide, glibornuride, tolbutamide, tolazamide, glipizide, carbutamide, gliquidone, glyhexamide, phenbutamide, tolcyclamide; glimepiride and gliclazide. Tolbutamide, glibenclamide, gliclazide, glibornuride, gliquidone, glisoxepid and glimepiride can be administered in the form that they are marketed under the trademarks RASTINON HOECHST™, AZUGLUCON™, DIAMICRONT™, GLUBORID™, GLURENORM™, PRO-DIABAN™ and AMARYL™, respectively.

Examples of GLP-1 agonists include, but are not limited to those disclosed in U.S. Patent Nos. 5,120,712, 5,118,666 and 5,512,549, and PCT Publication No. WO 91/11457. In particular, GLP-1 agonists include those compounds like GLP-1 (7-37) in which compound the carboxy-terminal amide functionality of Arg³⁶ is displaced with Gly at the 37^{th} position of the GLP-1 (7-36)NH₂ molecule and variants and analogs thereof including GLN⁹-GLP-1 (7-37), D-GLN⁹-GLP-1 (7-37), acetyl LYS⁹-GLP-1 (7-37), LYS¹⁸-GLP-1 (7-37) and, in particular, GLP-1 (7-37)OH, VAL⁸-GLP-1 (7-37), GLY⁸-GLP-1(7-37), THR⁸-GLP-1 (7-37), GLP-1 (7-37) and 4-imidazopropionyl-GLP-1.

One particular example of a GLP-1 agonist is exenatide, a 39-amino acid peptide amide, which is marketed under the trademark BYETTA™. Exenatide has the empirical formula C₁₈₄H₂₈₂N₅₀O₆₀S and molecular weight of 4186.6 Daltons. The amino acid sequence for Exenatide is as follows: H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH₂

Examples of beta-cell imidazoline receptor antagonists include, but are not limited to those described in PCT Publication No. WO 00/78726 and J. Pharmacol. Exp. Ther. 1996; 278; 82-89.

An example of an antidiabetic phenylacetic acid derivative is repaglinide and pharmaceutically acceptable salts thereof.

Examples of antidiabetic D-phenylalanine derivatives include, but are not limited to nateglinide (N-[(trans4-isopropylcyclohexyl)-carbonyl]-D-phenylalanine, EP 196222 and EP 526171) and repaglinide ((S)-2-ethoxy-4-{2-[[3-methy-1-1-[2-(1-piperidinyl)phenyl]butyl]-amino]-2-oxoethyl}benzoic acid, EP 0 147 850 A2 and EP 0 207 331 A1). Nateglinide is intended to include the particular crystal forms (polymorphs) disclosed in U.S. Patent No. 5,488,510 and European Patent Publication No. EP 0526171 B1. Repaglinide and nateglinide may be administered in the form as they are marketed under the trademarks NOVONORM™ and STARLIX™, respectively.

Examples of alpha-Glucosidase inhibitors include, but are not limited to, acarbose, N-(1,3-dihydroxy-2-propyl)valiolamine (voglibose) and the 1-deoxynojirimycin derivative miglitol. Acarbose is 4",6"-dideoxy-4'-[(1S)-(1,4,6/5)-4,5,6-trihydroxy-3-hydroxymethyl-2-cyclo-hexenylamino)maltotriose. The structure of acarbose can as well be described as O-4,6-dideoxy-4-{[1S,4R,5S,6S]-4,5,6-trihydroxy-3-(hydroxymethyl)-2-cyclohexen-1-yl]-amino)-alpha-D-glucopyranosyl-(1-4)-O-alpha-D-glucopyranosyl-(1-4)-D-glucopyranose. (U.S. Patent No. 4,062,950 and European Patent Publication No. EP 0 226 121). Acarbose and miglitol may be administered in the forms that they are marketed under the trademarks GLUCOBAY™ and DIASTABOL 50™ respectively.

Examples of inhibitors of gastric emptying other than GLP-1 include, but are not limited to those disclosed in J. Clin. Endocrinol. Metab. 2000, 85(3), 1043-1048, and Diabetes Care 1998; 21; 897-893, especially Amylin and analogs thereof such as pramlintide. Amylin is described in Diabetologia 39, 1996, 492-499.

Examples of α₂-adrenergic antagonists include, but are not limited to midaglizole which is described in Diabetes 36,1987, 216-220. The insulin that may be used in combination with Compound I include, but are not limited to animal insulin preparations extracted from the pancreas of bovine and pig; human insulin preparations genetically synthesized using *Escherichia coli* or yeast; zinc insulin; protamine zinc insulin; fragment or derivative of insulin (e.g., INS-1) and an oral insulin preparation.

In one particular embodiment, the antidiabetic compound administered in combination with Compound I is selected from the group consisting of nateglinide, mitiglinide, repaglinide, metformin, exenatide, rosiglitazone, tesaglitazar, pioglitazone, glisoxepid, glyburide, glibenclamide, acetohexamide, chloropropamide, glibornuride, tolbutamide, tolazamide, glipizide, carbutamide, gliquidone, glyhexamide, phenbutamide, tolcyclamide, glimepiride and gliclazide, including any pharmaceutically acceptable salts thereof.

Examples of the preparation and formulation of PTPase inhibitors, GSK-3 inhibitors, non-small molecule mimetic compounds, GFAT inhibitors, G6Pase inhibitors, glucagon receptor antagonists, PEPCK inhibitors, F-1,6-BPase inhibitors, GP inhibitors, RXR modulators, Beta-3 AR agonists, PDHK inhibitors, inhibitors of gastric emptying and UCP modulators are disclosed in the patents, applications and references provided herein.

In the case of combination therapy with Compound I, the other antidiabetic compound may be administered (e.g., route and dosage form) in a manner known per se for such compound. Compound I and the other antidiabetic compound may be administered sequentially (i.e., at separate times) or at the same time, either one after the other separately in two separate dose forms or in one combined, single dose form. In one particular embodiment, the other antidiabetic compound is administered with Compound I as a single, combined dosage form. The dose of the antidiabetic compound may be selected from the range known to be clinically employed for such compound. Any therapeutic compounds of diabetic complications, antihyperlipemic compounds, antiobestic compounds or antihypertensive compounds can be used in combination with Compound I in the same manner as the above antidiabetic compounds. Examples of therapeutic compounds of diabetic complications include, but are not limited to, aldose reductase inhibitors such as tolrestat, epalrestat, zenarestat, zopolrestat, minalrestat, fidarestat, CT-112 and ranirestat; neurotrophic factors and increasing compounds thereof such as NGF, NT-3, BDNF and neurotrophin production-secretion promoters described in WO01/14372 (e.g., 4-(4-chlorophenyl)-2-(2-methyl-1-imidazolyl)-5-[3-(2-methylphenoxy)propyl]oxazole); neuranagenesis stimulators such as Y-128; PKC inhibitors such as ruboxistaurin mesylate; AGE inhibitors such as ALT946, pimagedine, N-phenacylthiazolium bromide (ALT766), ALT-711, EXO-226, pyridorin and pyridoxamine; reactive oxygen scavengers such as thioctic acid; cerebral vasodilators such as tiapride and mexiletine; somatostatin receptor agonists such as BIM23190; and apoptosis signal regulating kinase-1 (ASK-1) inhibitors. Examples of antihyperlipemic compounds include, but are not limited to, HMG-CoA reductase inhibitors such as pravastatin, simvastatin, lovastatin, atorvastatin, fluvastatin, rosuvastatin and pitavastatin; squalene synthase inhibitors such as compounds described in WO97/10224 (e.g., N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid); fibrate compounds such as bezafibrate, clofibrate, simfibrate and clinofibrate; ACAT inhibitors such as avasimibe and eflucimibe; anion exchange resins such as colestyramine; probucol; nicotinic acid drugs such as nicomol and niceritrol; ethyl icosapentate; and plant sterols such as soysterol and γ-oryzanol. Examples of antiobestic compounds include, but are not limited to, dexfenfluramine, fenfluramine, phentermine, sibutramine, amfepramone, dexamphetamine, mazindol, phenylpropanolamine, clobenzorex; MCH receptor antagonists such as SB-568849 and SNAP-7941; neuropeptide Y antagonists such as CP-422935; cannabinoid receptor antagonists such as SR-141716 and SR-147778; ghrelin antagonist; 11β-hydroxysteroid dehydrogenase inhibitors such as BVT-3498; pancreatic lipase inhibitors such as orlistat and ATL-962; Beta-3 AR agonists such as AJ-9677; peptidic anorexiants such as leptin and CNTF (Ciliary Neurotropic Factor); cholecystokinin agonists such as lintitript and FPL-15849; and feeding deterrent such as P-57. Examples of the antihypertensive compounds include angiotensin converting enzyme inhibitors such as captopril, enalapril and delapril; angiotensin II antagonists such as candesartan cilexetil, losartan, eprosartan, valsartan, telmisartan, irbesartan, olmesartan medoxomil, tasosartan and 1-[[2'-(2,5-dihydro-5-oxo-4H-1,2,4-oxadiazol-3-yl)biphenyl-4-yl]methyl]-2-ethoxy-1H-benzimidazole-7-carboxylic acid; calcium channel blockers such as manidipine, nifedipine, nicardipine, amlodipine and efonidipine; potassium channel openers such as levcromakalim, L-27152, AL0671 and NIP-121; and clonidine.

The structure of the active agents identified herein by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active agents and, based on these references, likewise enabled to manufacture and test the pharmaceutical indications and properties in standard test models, both in vitro and in vivo.

### 4. COMPOSITIONS COMPRISING COMPOUND I

Compound I (dosed as defined in the claims) may be comprised within a pharmaceutical composition adapted for a variety of routes of administration. For example, Compound I may be comprised within a pharmaceutical composition adapted to be administered by a route selected from the group consisting of orally, parenterally, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly, intraperitoneally and intrathecally. As such, Compound I may be formulated in a variety of pharmaceutically acceptable compositions including injectable forms (e.g. subcutaneous, intravenous, intramuscular and intraperitoneal injections), drip infusions, external application forms (e.g. nasal spray preparations, transdermal preparations; ointments, etc.), and suppositories (e.g. rectal and vaginal suppositories). These different pharmaceutically acceptable compositions can be manufactured by known techniques conventionally used in the pharmaceutical industry with a pharmaceutically acceptable carrier conventionally used in the pharmaceutical industry.

As used herein, a composition comprising Compound I is intended to encompass the free base form of Compound I, salts, hydrates and prodrugs of Compound I, as well as other materials that may be included in such composition for its intended purpose, including other active ingredients, unless otherwise specified. Particular salt forms of Compound I that may be employed include, but are not limited to, the benzoate, toluenesulfonate and hydrochloride salt forms.
According to the present invention Compound I is formulated in a single dose form between 5 mg and 250 mg of Compound I to a patient wherein Compound I is present in the pharmaceutical composition in a benzoate salt, with the exception of the following compositions: (a) a pharmaceutical composition formulated for oral administration consisting of 10 to 100 mg of Compound I, 105 mg of citric acid monohydrate, 18 mg of sodium hydroxide, a flavouring and water to 100 ml; and (b) a pharmaceutical composition formulated for intravenous administration consisting of 5 to 10 mg of Compound I, 1.05 mg of citric acid monohydrate, 0.18 mg of sodium hydroxide, a sufficient quantity of dextrose monohydrate to make the formulation isotonic, and water to 1 ml. Also according to the present invention Compound I is formulated in a single dose form comprising 12.5 mg, 20 mg, 25 mg, 50 mg, or 75 mg Compound I. As also noted above, it is desirable for Compound I to be administered one time per day.

As also noted above, Compound I may advantageously be used when administered orally. Accordingly, the compositions of the present invention may optionally be adapted for oral administration. In one variation, such pharmaceutical composition is a solid formulation adapted for oral administration. In this regard, the composition, for example, may be in the form of a tablet or capsule. Example 2 provides examples of solid formulations comprising Compound I adapted for oral administration. In another variation, such pharmaceutical composition is a liquid formulation adapted for oral administration.

As noted above, Compound I may advantageously be used in combination with one or more other antidiabetic compounds. Accordingly, the compositions of the present invention may optionally comprises Compound I in combination with one or more other antidiabetic compounds in a combined, single dose form.

Optionally, such combined, single dose form comprising Compound I in combination with one or more other antidiabetic compounds is adapted for oral administration and optionally is a solid oral dose form.

In one variation, such combined, single dose form comprising Compound I in combination with one or more other antidiabetic compounds comprises between 5 mg and 250 mg of Compound I to a patient, optionally between 10 mg and 200 mg of Compound I, optionally between 10 mg and 150 mg of Compound I, and optionally between 10 mg and 100 mg of Compound I. (in each instance based on the molecular weight of the free base form of Compound I). In specific embodiments, such combined, single dose form comprising Compound I in combination with one or more other antidiabetic compounds comprises 10 mg, 12.5 mg, 20 mg, 25 mg, 50 mg, 75 mg, and 100 mg of Compound I.

Any antidiabetic compound, or set of antidiabetic compounds may be combined with Compound I to form such combined, single dose form. In particular embodiments, such combined, single dose form includes Compound I and one or more members of the group consisting of insulin signaling pathway modulators, like protein tyrosine phosphatase (PTPase) inhibitors, and glutamine-fructose-6-phosphate amidotransferase (GFAT) inhibitors, compounds influencing a dysregulated hepatic glucose production, like glucose-6-phosphatase (G6Pase) inhibitors, fructose-1,6-bisphosphatase (F-1,6-BPase) inhibitors, glycogen phosphorylase (GP) inhibitors, glucagon receptor antagonists and phosphoenolpyruvate carboxykinase (PEPCK) inhibitors, pyruvate dehydrogenase kinase (PDHK) inhibitors, insulin sensitivity enhancers (insulin sensitizers), insulin secretion enhancers (insulin secretagogues), alpha-glucosidase inhibitors, inhibitors of gastric emptying, glucokinase activators, GLP-1 receptor agonists, UCP modulators, RXR modulators, GSK-3 inhibitors, PPAR modulators, metformin, insulin, and α₂-adrenergic antagonists. Compound I may be administered with such at least one other antidiabetic compound either simultaneously as a single dose, at the same time as separate doses, or sequentially (i.e., where on is administered before or after the other is administered).

In one variation, such combined, single dose form comprises Compound I and an antidiabetic thiazolidinedione. Particular examples of thiazolidinediones that may be used in this variation include, but are not limited to (S)-((3,4-dihydro-2-(phenyl-methyl)-2H-1-benzopyran-6-yl)methyl-thiazolidine-2,4-dione (englitazone), 5-{[4-(3-(5-methyl-2-phenyl-4-oxazolyl)-1-oxo-propyl)-phenyl]-methyl}-thiazolidine-2,4-dione (darglitazone), 5-{[4-(1-methyl-cyclohexyl)methoxy)-phenyl]methyl]-thiazolidine-2,4-dione (ciglitazone), 5-{[4-(2-(1-indolyl)ethoxy)phenyl]methyl}-thiazolidine-2,4-dione (DRF2189), 5-{4-[2-(5-methyl-2-phenyl-4-oxazoly)-ethoxy)]benzyl}-thiazolidine-2,4-dione (BM-13.1246), 5-(2-naphthylsulfonyl)-thiazolidine-2,4-dione (AY-31637), bis{4-[(2,4-dioxo-5-thiazolidinyl)-methyl]phenyl}methane (YM268), 5-{4-[2-(5-methyl-2-phenyl-4-oxazolyl)-2-hydroxyethoxy]-benzyl}-thiazolidine-2,4-dione (AD-5075), 5-[4-(1-phenyl-1-cyclopropanecarbonylamino)-benzyl]-thiazolidine-2,4-dione (DN-108) 5-{[4-(2-(2,3-dihydroindol-1-yl)ethoxy)phenylmethyl)-thiazolidine-2,4-dione, 5-[3-(4-chloro-phenyl])-2--propynyl]-5-phenylsulfonyl)thiazolidine-2,4-dione, 5-[3-(4-chlorophenyl])-2-propynyl]-5-(4-fluorophenyl-sulfonyl)thiazolidine-2,4-dione, 5-{[4-(2-(methyl-2-pyridinyl-amino)-ethoxy)phenyl]methyl}-thiazolidine-2,4-dione (rosiglitazone), 5-{[4-(2-(5-ethyl-2-pyridyl)ethoxy)phenyl]-methyl}-thiazolidine-2,4-dione (pioglitazone), 5-[6-(2-fluoro-benzyloxy)-naphthalen-2-ylmethyl]-thiazolidine-2,4-dione (MCC555), 5-([2-(2-naphthyl)-benzoxazol-5-yl]-methyl}thiazolidine-2,4-dione (T-174), edaglitazone (BM-13-1258), rivoglitazone (CS-011) and 5-(2,4-dioxothiazolidin-5-ylmethyl)-2-methoxy-N-(4-trifluoromethyl-benzyl)benzamide (KRP297).

In one particular variation, the thiazolidinedione in such combined, single dose form is 5-{[4-(2-(5-ethyl-2-pyridyl)ethoxy)phenyl]-methyl}-thiazolidine-2,4-dione (pioglitazone) and its hydrochloride salt which is marketed under the trademark ACTOS™.

In another particular variation, the thiazolidinedione is 5-{[4-(2-(methyl-2-pyridinyl-amino)-ethoxy)phenyl]methyl}-thiazolidine-2,4-dione (rosiglitazone) and its maleate salt.

In another variation, such combined, single dose form comprises Compound I and a non-glitazone type PPAR gamma agonist.

In another variation, such combined, single dose form comprises Compound I and a biguanide. A particular example of a biguanide that may be used in this variation is Metformin (dimethyldiguanide) and its hydrochloride salt which is marketed under the trademark GLUCOPHAGE™.

In another variation, such combined, single dose form comprises Compound I and a sulphonyl urea derivative. Particular examples of sulphonyl urea derivatives that may be used in this variation include, but are not limited to glisoxepid, glyburide, glibenclamide, acetohexamide, chloropropamide, glibornuride, tolbutamide, tolazamide, glipizide, carbutamide, gliquidone, glyhexamide, phenbutamide, tolcyclamide; glimepiride and gliclazide. Tolbutamide, glibenclamide, gliclazide, glibornuride, gliquidone, glisoxepid and glimepiride can be administered in the form as they are marketed under the trademarks RASTINON HOECHST™, AZUGLUCON™, DIAMICRONT™, GLUBORID™, GLURENORM™, PRO-DIABAN™ and AMARYL™, respectively.

In another variation, such combined, single dose form comprises Compound I and an antidiabetic D-phenylalanine derivative. Particular examples of antidiabetic D-phenylalanine derivatives that may be used in this variation include, but are not limited to repaglinide and nateglinide which may be administered in the form as they are marketed under the trademarks NOVONORM™ and STARLIX™, respectively.

In another variation, such combined, single dose form comprises Compound I and an alpha-Glucosidase inhibitor. Particular examples of alpha-Glucosidase inhibitors that may be used in this variation include, but are not limited to acarbose, miglitol and voglibose which may be administered in the form as they are marketed under the trademarks GLUCOBAY™, DIASTABOL 50™ and BASEN™, respectively.

In one particular embodiment, the antidiabetic compound administered in combination with Compound I in such combined, single dose form is selected from the group consisting of nateglinide, mitiglinide, repaglinide, metformin, exenatide, rosiglitazone, pioglitazone, glisoxepid, glyburide, glibenclamide, acetohexamide, chloropropamide, glibornuride, tolbutamide, tolazamide, glipizide, carbutamide, gliquidone, glyhexamide, phenbutamide, tolcyclamide, glimepiride and gliclazide, including any pharmaceutically acceptable salts thereof.

In regard to each of the above embodiments and variations regarding a combined, single dose form comprising the combination of Compound I and one or more other antidiabetic compounds, the pharmaceutical composition may optionally be adapted for oral administration and in this regard may optionally be a solid formulation such as a tablet or capsule or may alternatively be in a liquid formulation adapted for oral administration. The dose of the antidiabetic compound may be selected from the range known to be clinically employed for such compound. Any therapeutic compounds of diabetic complications, antihyperlipemic compounds, antiobestic compounds or antihypertensive compounds can be used in combination with Compound I in the same manner as the above antidiabetic compounds. Examples of therapeutic compounds of diabetic complications include, but are not limited to, aldose reductase inhibitors such as tolrestat, epalrestat, zenarestat, zopolrestat, minalrestat, fidarestat, CT-112 and ranirestat; neurotrophic factors and increasing compounds thereof such as NGF, NT-3, BDNF and neurotrophin production-secretion promoters described in WO01/14372 (e.g., 4-(4-chlorophenyl)-2-(2-methyl-1-imidazolyl)-5-[3-(2-methylphenoxy)propyl]oxazole); neuranagenesis stimulators such as Y-128; PKC inhibitors such as ruboxistaurin mesylate; AGE inhibitors such as ALT946, pimagedine, N-phenacylthiazolium bromide (ALT766), ALT-711, EXO-226, pyridorin and pyridoxamine; reactive oxygen scavengers such as thioctic acid; cerebral vasodilators such as tiapride and mexiletine; somatostatin receptor agonists such as BIM23190; and apoptosis signal regulating kinase-1 (ASK-1) inhibitors. Examples of antihyperlipemic compounds include, but are not limited to, HMG-CoA reductase inhibitors such as pravastatin, simvastatin, lovastatin, atorvastatin, fluvastatin, rosuvastatin and pitavastatin; squalene synthase inhibitors such as compounds described in WO97/10224 (e.g., N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid); fibrate compounds such as bezafibrate, clofibrate, simfibrate and clinofibrate; ACAT inhibitors such as avasimibe and eflucimibe; anion exchange resins such as colestyramine; probucol; nicotinic acid drugs such as nicomol and niceritrol; ethyl icosapentate; and plant sterols such as soysterol and γ-oryzanol. Examples of antiobestic compounds include, but are not limited to, dexfenfluramine, fenfluramine, phentermine, sibutramine, amfepramone, dexamphetamine, mazindol, phenylpropanolamine, clobenzorex; MCH receptor antagonists such as SB-568849 and SNAP-7941; neuropeptide Y antagonists such as CP-422935; cannabinoid receptor antagonists such as SR-141716 and SR-147778; ghrelin antagonist; 11β-hydroxysteroid dehydrogenase inhibitors such as BVT-3498; pancreatic lipase inhibitors such as orlistat and ATL-962; Beta-3 AR agonists such as AJ-9677; peptidic anorexiants such as leptin and CNTF (Ciliary Neurotropic Factor); cholecystokinin agonists such as lintitript and FPL-15849; and feeding deterrent such as P-57. Examples of the antihypertensive compounds include angiotensin converting enzyme inhibitors such as captopril, enalapril and delapril; angiotensin II antagonists such as candesartan cilexetil, losartan, eprosartan, valsartan, telmisartan, irbesartan, olmesartan medoxomil, tasosartan and 1-[[2'-(2,5-dihydro-5-oxo-4H-1,2,4-oxadiazol-3-yl)biphenyl-4-yl]methyl]-2-ethoxy-1H-benzimidazole-7-carboxylic acid; calcium channel blockers such as manidipine, nifedipine, nicardipine, amlodipine and efonidipine; potassium channel openers such as levcromakalim, L-27152, AL0671 and NIP-121; and clonidine.

### 5. KITS AND ARTICLES OF MANUFACTURE COMPRISING COMPOUND I

The present invention also relates to kits comprising a pharmaceutical composition according to the present invention comprising Compound I (and optionally one or more other antidiabetic compounds) in the dosage as defined in the claims where such kit further comprises instructions that include one or more forms of information selected from the group consisting of indicating a disease state for which the pharmaceutical composition is to be administered, storage information for the pharmaceutical composition, dosing information and instructions regarding how to administer the pharmaceutical composition. The kit may also comprise packaging materials. The packaging material may also comprise a container for housing the pharmaceutical composition. The container may optionally comprise a label indicating the disease state for which the pharmaceutical composition is to be administered, storage information, dosing information and/or instructions regarding how to administer the composition. The kit may also comprise additional components for storage or administration of the composition. The kit may also comprise the composition in single or multiple dose forms.

In one embodiment, the pharmaceutical composition in the kit comprises multiple doses of a pharmaceutical composition according to the present invention wherein such pharmaceutical composition is a single dose form that comprises Compound I in one of the dosage ranges specified herein.

In another embodiment, the pharmaceutical composition in the kit comprises multiple doses of a pharmaceutical composition according to the present invention wherein such pharmaceutical composition is a single dose form that comprises Compound I and one or more of the other antidiabetic compounds specified herein.

The present invention also relates to articles of manufacture comprising a pharmaceutical composition according to the present invention comprising Compound I (and optionally one or more other antidiabetic compounds) where such articles of manufacture further comprise packaging materials. In one variation, the packaging material comprises a container for housing the composition. In another variation, the invention provides an article of manufacture where the container comprises a label indicating one or more members of the group consisting of a disease state for which the composition is to be administered, storage information, dosing information and/or instructions regarding how to administer the composition.

In one embodiment, the pharmaceutical composition in the article of manufacture comprises multiple doses of a pharmaceutical composition according to the present invention wherein such pharmaceutical composition is a single dose form that comprises Compound I in one of the dosage ranges specified herein.

In another embodiment, the pharmaceutical composition in the article of manufacture comprises multiple doses of a pharmaceutical composition according to the present invention wherein such pharmaceutical composition is a single dose form that comprises Compound I and one or more of the other antidiabetic compounds specified herein.

It is noted that the packaging material used in kits and articles of manufacture according to the present invention may form a plurality of divided containers such as a divided bottle or a divided foil packet. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. The container that is employed will depend on the exact dosage form involved. It is feasible that more than one container can be used together in a single package to market a single dosage form. For example, tablets may be contained in a bottle that is in turn contained within a box.

One particular example of a kit according to the present invention is a so-called blister pack. Blister packs are well known in the packaging industry and are being widely used for the packaging of pharmaceutical unit dosage forms (tablets, capsules, and the like). Blister packs generally consist of a sheet of relatively stiff material (preferably stiff transparent plastic material) covered with a foil. During the packaging process recesses are formed in the stiff material. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the foil and the sheet. The strength of the sheet is preferably such that the tablets or capsules can be removed from the blister pack by manually applying pressure on the recesses whereby an opening is formed in the foil at the place of the recess. The tablet or capsule can then be removed via said opening.

### EXAMPLES

### 1. Preparation of 2-[[6-[(3R)-3-amino-1-piperidinyl]-3,4-dihydro-3-methyl-2,4-dioxo-1(2H)-pyrimidinyl]methyl]-benzonitrile and pharmaceutically acceptable salts

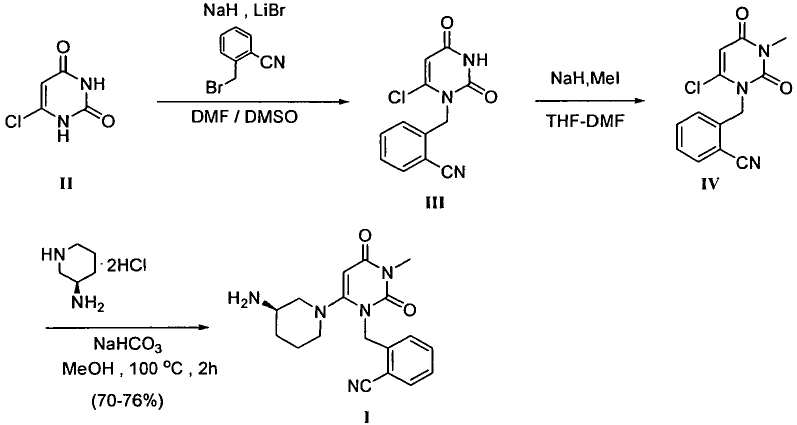

### Compound III

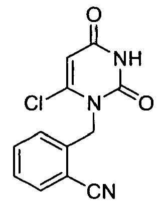

**2-(6-Chloro-2,4-dioxo-3,4-dihydro-2H-pyrimidin-1-ylmethyl)-benzonitrile (III).** To a solution of 6-chlorouracil (20 g, 122 mmol) in a mixture of DMF-DMSO (6:1, 600 mL) under nitrogen at 0 °C, was added sodium hydride (60%, 5.5 g, 137 mmol) in portions. After 0.5h, lithium bromide (8 g, 96 mmol) was added into the mixture and stirred for 15 min at 0 °C. A solution of *α*-Bromo-*o*-tolunitrile (25.1 g, 128 mmol) in DMF (30 mL) was added dropwise, and stirred at this temperature for 1 h, and then RT overnight. The mixture was evaporated and co-evaporated with water *in vacuo* to remove most of DMF, and then poured into ice water (1L). The precipitate was collected by filtration. The crude product was suspended in hot AcOEt-CHCl₃ and sonicated for 5 min, allowed to stand at 0 °C for 1h, and then filtered to give a white solid of the title compound (19 g) in 54% yield. ¹H-NMR (400 MHz, DMSO): δ 11.82 (s, 1H), 7.87 (d, 1H, J = 7.6 Hz), 7.71 (t, 1H, J = 7.6 Hz), 7.51 (t, 1H, J = 7.6 Hz), 7.37 (d, 1H, J = 8 Hz), 6.06 (s, 1H), 5.31 (s, 2H). MS (ES) [m+H] calc'd for C₁₂H₉ClN₃O₂, 262.0; found 262.0.

### Compound IV

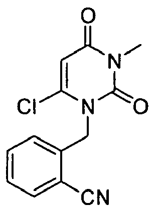

**2-(6-Chloro-3-methyl-2,4-dioxo-3,4-dihydro-2H-pyrimidin-1-ylmethyl)-benzonitrile (IV).** To a cold (0 °C) solution of benzylated 6-chlorouracil **III** (10 g, 38 mmol) in DMF-THF (1:1, 300 mL) under nitrogen, was added NaH (60%, 1.6 g, 39.9 mmol) in portions, followed by adding LiBr (2g). The mixture was stirred at r.t for 20 min. After adding iodomethane (5.4 mL, 76 mmol), the flask was sealed and stirred at this temperature for 10 min, rt for 2h, and 35 °C overnight, and then concentrated *in vacuo.* The residue was dissolved in CHCl₃ and washed with water and brine, dried (Na₂SO₄), and filtered then concentrated *in vacuo.* The crude product was crystallized from THF-Hexanes to give 7.6 g (72%) of the title compound **IV.** ¹H NMR (400 MHz, DMSO): δ 7.87 (d, 1H, J = 7.6 Hz), 7.70 (t, 1H, J = 7.6 Hz), 7.51 (t, 1H, J = 7.6 Hz), 7.40 (d, 1H, J = 8 Hz), 6.21 (s, 1H), 5.38 (s, 2H), 3.28 (s, 3H). MS (ES) [m+H] calc'd for C₁₃H₁₁ClN₃O₂, 276.1; found 276.1.

### Compound I (TFA Salt)

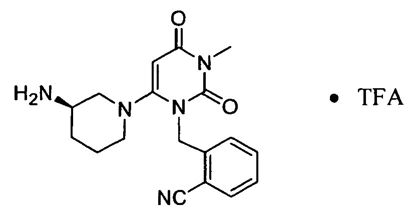

**2-[[6-[(3R)-3-amino-1-piperidinyl]-3,4-dihydro-3-methyl-2,4-dioxo-1(2H)-pyrimidinyl]methyl]-benzonitrile (I).** 2-(6-Chloro-3-methyl-2,4-dioxo-3,4-dihydro-2-H-pyrimidin-1-ylmethyl)-benzonitrile (330 mg, 1.08 mmol), (*R*)-3-amino-piperidine dihydrochloride (246 mg, 1.4 mmol) and sodium bicarbonate (500 mg, 5.4 mmol) were stirred with 200 mg activated molecular sieves (4A) in dry MeOH (5 mL) at 100 °C in a sealed tube for 2 h. The reaction was filtered through Celite, concentrated *in vacuo*, and then diluted with CHCl₃, and washed with water. The water phase was extracted with CHCl₃ and the combined organic phases were washed with water, dried (Na₂SO₄), and filtered. TFA (1mL) was added into the solution which was then concentrated *in vacuo.* The residue was dissolved in a small amount of MeOH, and Et₂O was added to force precipitation. The mixture was allowed to stand at RT overnight. Solvents were decanted, and the solid was washed with Et₂O two times to give 270 mg TFA salt of **Compound I** as off-white powder.

The TFA salt of **Compound I** has ¹H-NMR (400 MHz, CDCl₃-CD₃OD 10:1): δ 7.82 (d, 1H, J = 7.6 Hz), 7.65 (t, 1H, J = 7.6 Hz), 7.46 (t, 1H, J = 7.6 Hz), 7.23 (d, 1H, J = 8.0 Hz), 5.42 (s, 1H), 5.50-5.00 (ABq, 2H, J = 41.6, 15.2 Hz), 3.30 (m, 2H), 3.16 (s, 3H), 2.91 (m, 1H), 2.76 (m, 2H), 1.93 (m, 1H), 1.79 (m, 1H), 1.51 (m, 2H). MS (ES) [m+H] calc'd for C₁₈H₂₂N₅O₂, 340.2; found, 340.2.

It will be understood by those skilled in the art that condensation with the amine or amine hydrochloride may be performed in a solvent or mixture of solvents with a base, such as potassium carbonate, sodium bicarbonate and the like, or mixtures thereof. The solvent may comprise both protic and aprotic solvents, or mixtures thereof. For example, the solvent may comprise a mixture of isopropyl alcohol and water. It will also be understood that the product may be further purified by washing with an organic solvent or mixture of solvents. Non-limiting examples of solvent or solvent mixtures include isopropyl acetate, ethyl acetate, dichloromethane, heptane, and the like. Further, the product may optionally be purified by column chromatography.

The benzonitrile product may be isolated as the free base if desired, but preferably, the product may be further converted to a corresponding acid addition salt. For example, the benzoic acid salt was formed by treating the benzonitrile product with benzoic acid to form 2-[6-(3-amino-piperidin-1-yl)-3-methyl-2,4-dioxo-3,4-dihydro-2H-pyrimidin-1-ylmethyl]-benzonitrile benzoate (**I**). Preparation and isolation of the benzoate salt was performed by conventional methods for the formation of acid addition salts. ¹H-NMR (400 MHz, CDCl₃-CD₃OD 10:1): δ 7.82 (d, 1H, J = 7.6 Hz), 7.65 (t, 1H, J = 7.6 Hz), 7.46 (t, 1H, J = 7.6 Hz), 7.23 (d, 1H, J = 8.0 Hz), 5.42 (s, 1H), 5.50-5.00 (ABq, 2H, J = 41.6, 15.2 Hz), 3.30 (m, 2H), 3.16 (s, 3H), 2.91 (m, 1H), 2.76 (m, 2H), 1.93 (m, 1H), 1.79 (m, 1H), 1.51 (m, 2H). MS (ES) [m+H] calc'd for C₁₈H₂₂N₅O₂, 340.2; found, 340.2.

Following the same procedure described above, HCl addition salt was prepared as follows. A free base form of **I** was isolated after the crude product was washed with water, dried over Na₂SO₄, filtered and concentrated. The free base product was then dissolved in THF. Alternatively, the free base could be dissolved in other solvents, such as dioxane, acetonitrile, ethyl acetate, dichloromethane, etc., or mixtures thereof. The solution was then stirred and 1.2 equivalents of 4M HCl in dioxane was added dropwise. After 10 min stirring, the suspended mixture was allowed to stand at rt for 1h, and then filtered to give the solid HCl salt form of **I.** ¹H-NMR (400 MHz, DMSO-D6): δ 7.82 (d, 1H, J = 7.6 Hz), 7.65 (t, 1H, J = 7.6 Hz), 7.46 (t, 1H, J = 7.6 Hz), 7.23 (d, 1H, J = 8.0 Hz), 5.42 (s, 1H), 5.20, 5.08 (ABq, 2H, J = 41.6, 15.2 Hz), 3.30 (m, 2H), 3.16 (s, 3H), 2.91 (m, 1H), 2.76 (m, 2H), 2.50 (bs, 2 H), 1.93 (m, 1H), 1.79 (m, 1H), 1.51 (m, 2H). MS (ES) [m+H] calc'd for C₁₈H₂₂N₅O₂, 340.2; found, 340.2.

Further, the toluenesulfonate salt was prepared as follows. A 200µL aliquot of a 0.03M stock solution of free base was dissolved in dichoromethane and concentrated under a slow stream of nitrogen. The resulting free base was dissolved in 150 µL of solvent (*e.g.*, acetic acid, acetone, ethanol, THF or dichloromethane) and the solution shaken for 10 minutes. The shaken solution was then charged with 50 µL of a 0.126M solution of touenesulfonic acid (1.05 eq.) in dioxane. The solution was shaken for 3 hours, followed by removal of the solvents under a stream of nitrogen to provide the toluenesulfonate salt.

The toluenesulfonate salt was also prepared by dissolving 2g of the free base in 10 volumes of acetonitrile and heating the solution to 75 °C for 10 minutes. Then p-toluenesulfonic acid (1.05 equivalents) was added and the solution held at 75 °C for 5 minutes. The temperature was ramped down (at about 25°C/hr) and stirred at room temperature overnight. The product (2.64 g) was dried in a vacuum oven at 50°C and 698.5 mm Hg with a nitrogen sweep for 18 hours.

The isolation and/or purification steps of the intermediate compounds in the above described process may optionally be avoided if the intermediates from the reaction mixture are obtained as relatively pure compounds and the by-products or impurities of the reaction mixture do not interfere with the subsequent reaction steps. Where feasible, one or more isolation steps may be eliminated to provide shorter processing times, and the elimination of further processing may also afford higher overall reaction yields.

### 2. Exemplary formulations comprising benzoate salt of 2-{6-[3(R)-Amino-piperidin-1-yl]-3-methyl-2,4-dioxo-3,4-dihydro-2H-pyrimidin-1-ylmethyl}-benzonitrile

Provided are examples of tablet formulations that may be used to administer benzoate salt of 2-[[6-[(3R)-3-amino-1-piperidinyl]-3,4-dihydro-3-methyl-2,4-dioxo-1(2H)-pyrimidinyl]methyl]-benzonitrile (benzoate salt) (Compound I) according to the present invention. It is noted that the formulations provided herein may be varied as is known in the art.

The exemplary tablet formulations are as follows:

### 12.5mg of Compound I (weight of free base form) per tablet

**Core Tablet Formulation**

| | |
|---|---|
| (1) 2-[[6-[(3R)-3-amino-1-piperidinyl]-3,4-dihydro-3-methyl-2,4-dioxo-1(2H)-pyrimidinyl]methyl]-benzonitrile (benzoate salt) | 17.0 mg |
| (2) Lactose Monohydrate, NF, Ph, Eur (FOREMOST 316 FAST FLO) | 224.6 mg |
| (3) Microcrystalline Cellulose, NF, Ph, Eur (AVICEL PH 102) | 120.1 mg |
| (4) Croscarmellose Sodium, NF, Ph, Eur (AC-DI-SOL) | 32.0 mg |
| (5) Colloidal Silicon Dioxide, NF, Ph, Eur (CAB-O-SIL M-5P) | 3.2 mg |
| (6) Magnesium Stearate, NF, Ph, Eur (MALLINCKRODT, Non-bovine Hyqual) | 3.2 mg |
| TOTAL (per tablet) | 400.0 mg |
| Film Coat (12.0 mg in total) | |

| | |
|---|---|
| (1) Opadry II 85F18422, White - Portion 1 (COLORCON) (2) Opadry II 85F18422, White - Portion 2 (COLORCON) (3) Opadry II 85F18422, White - Portion 3 (COLORCON) | |

### 25mg of Compound I (weight of free base form) per tablet

**Core Tablet Formulation**

| | |
|---|---|
| (1) 2-[[6-[(3R)-3-amino-1-piperidinyl)-3,4-dihydro-3-methyl-2,4-dioxo-1(2H)-pyrimidinyl]methyl]-benzonitrile (benzoate salt) | 34.0 mg |
| (2) Lactose Monohydrate, NF, Ph, Eur (FOREMOST 316 FAST FLO) | 207.6 mg |
| (3) Microcrystalline Cellulose, NF, Ph, Eur (AVICEL PH 102) | 120.1 mg |
| (4) Croscarmellose Sodium, NF, Ph, Eur (AC-DI-SOL) | 32.0 mg |
| (5) Colloidal Silicon Dioxide, NF, Ph, Eur (CAB-O-SIL M-5P) | 3.2 mg |
| (6) Magnesium Stearate, NF, Ph, Eur (MALLINCKRODT, Non-bovine Hyqual) | 3.2 mg |
| TOTAL (per tablet) | 400.0 mg |
| Film Coat (12.0 mg in total) | |

| | |
|---|---|
| (1) Opadry II 85F18422, White - Portion 1 (COLORCON) (2) Opadry II 85F 18422, White - Portion 2 (COLORCON) (3) Opadry II 85F18422, White - Portion 3 (COLORCON) | |

### 50mg of Compound I (weight of free base form) per tablet

**Core Tablet Formulation**

| | |
|---|---|
| (1) 2-[[6-[(3R)-3-amino-1-piperidinyl]-3,4-dihydro-3-methyl-2,4-dioxo-1(2H)-pyrimidinyl]methyl]-benzonitrile (benzoate salt) | 68.0 mg |
| (2) Lactose Monohydrate, NF, Ph, Eur (FOREMOST 316 FAST FLO) | 173.6 mg |
| (3) Microcrystalline Cellulose, NF, Ph, Eur (AVICEL PH 102) | 120.1 mg |
| (4) Croscarmellose Sodium, NF, Ph, Eur (AC-DI-SOL) | 32.0 mg |
| (5) Colloidal Silicon Dioxide, NF, Ph, Eur (CAB-O-SIL M-5P) | 3.2 mg |
| (6) Magnesium Stearate, NF, Ph, Eur (MALLINCKRODT, Non-bovine Hyqual) | 3.2 mg |
| TOTAL (per tablet) | 400.0 mg |
| Film Coat (12.0 mg in total) | |

| | |
|---|---|
| (1) Opadry II 85F 18422, White - Portion 1 (COLORCON) (2) Opadry II 85F 18422, White - Portion 2 (COLORCON) (3) Opadry II 85F18422, White - Portion 3 (COLORCON). | |

### 6. EFFECT OF ADMINISTRATION ON PLASMA DPP-IV ACTIVITY

A double-blind, placebo-controlled, repeat-dose, multicenter study using 3 dose levels of Compound I was performed using 56 newly diagnosed type II diabetes patients. Patients were randomly assigned to 1 of 4 treatment groups (Compound I at 25 mg/day, 100 mg/day, or 400 mg/day, or placebo capsules). Compound I was administered for 14 days to the patients. Blood samples were collected on Days 6, 16, 17, and 21 for analysis of efficacy based on change in mean 4-hour postprandial plasma glucose (Cavg) from Day -1 to Day 14. Secondary efficacy endpoints included mean 4-hour prandial fructosamine, and glycosylated hemoglobin (HbA_{1c}). Data was collected at each study visit. Inhibition of DPPIV activity was also determined using an assay validated for human plasma samples.

### (a) Effect of Administration on Plasma Glucose Lowering by Compound I

The primary efficacy analysis was based on change in mean 4-hour prandial glucose concentrations (Cavg) from Day -1 to Day 14. Figure 1 provides a table summarizing the primary efficacy endpoint measured following breakfast. Following 14 days of treatment with Compound I, 4-hour prandial glucose concentrations following breakfast (Cavg B) for all Compound I groups were significantly reduced from baseline compared with placebo. Fourteen days of treatment with Compound I produced mean reductions from baseline in Cavg B of 33 mg/dL, 37 mg/dL, and 66 mg/dL for the 25 mg, 100 mg, and the 400-mg Compound I groups, respectively. When calculated as a percent change, mean reductions of 15%, 17%, and 24% were observed for the 25 mg, 100 mg, and the 400 mg Compound I groups, respectively.

### (b) Effect of Administration on Glycosylated Hemoglobin (HbA_{1c}) by Compound I

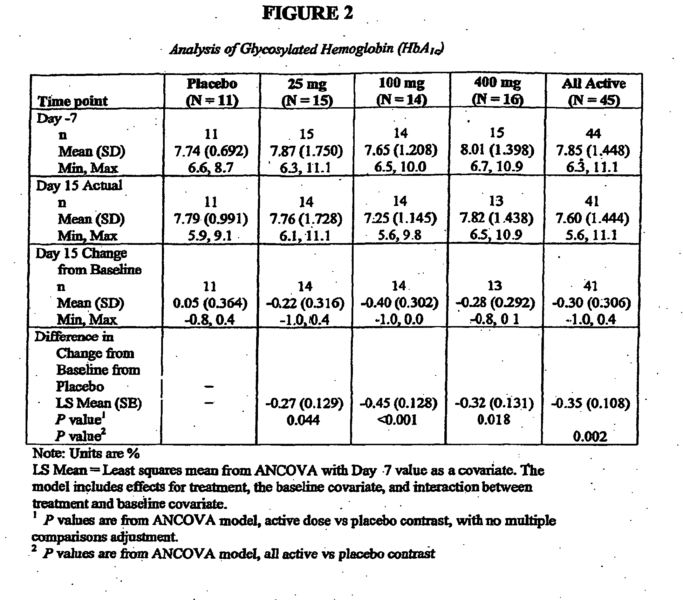

Figure 2 provides a table summarizing HbA_{1c} results by treatment and by time point. Mean values of HbA_{1c} were reduced from baseline following 14 days of treatment for all Compound I groups. The change from baseline for each of the Compound I groups was significantly different than placebo (*P*=0.044, *P*<0.001, and *P*=0.018 for the 25 mg, 100 mg, and 400 mg Compound I groups, respectively) as was the change from baseline for all Compound I treatment combined (*P*=0.002). The difference from placebo was greatest for the Compound I group that received the 100 mg dose.

### (c) Effect of Administration on Fasting Fructosamine Blood Concentrations by Compound I

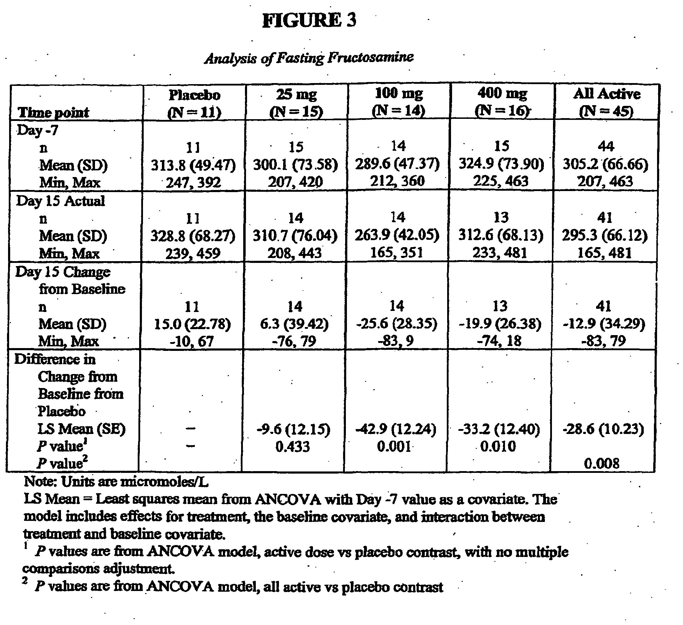

Figure 3 provides a table summarizing fasting fructosamine results by treatment and by time point. Fasting fructosamine was significantly decreased from baseline compared with placebo following 14 days of treatment with Compound I at 100 mg (*P*=0.001) and 400 mg (*P*=0.010). The change from baseline for all Compound I treatment combined was also significantly different than placebo (*P*=0.008). The difference from placebo was greatest for the 100 mg Compound I group, consistent with the analysis of HbA_{1c}.

### (d) Inhibition of Plasma DPP-IV Activity by Compound I

Figure 4 illustrates the observed effect that administering Compound I has on a patient's plasma DPPIV activity. As can be seen, peak inhibition of DPPIV activity following a single dose of Compound I exceeded 94% across all Compound I dose groups, with median time to peak inhibition ranging from 1 to 2 hours. After 14 days of once daily dosing, peak inhibition was similar to that observed on Day 1. Thus, as can be seen from the data shown in Figure 4, by administering Compound I one time per day at the dosage levels specified herein, Compound I can be effectively used relative to disease states where it is desired to reduce the patient's plasma DPPIV activity by greater than 60%, optionally greater than 70%, and optionally greater than 80%. Specifically, when at least 25mg of Compound I is administered, the patient's plasma DPPIV activity may be reduced by greater than 60% relative to baseline for a period of at least at least 6 hours, 12 hours, 18 hours and even 24 hours following administration.

### 7. EFFECT OF CO-ADMINISTRATION WITH PIOGLITAZONE ON GLYCOSYLATED HEMOGLOBIN

The effect of administering Compound I in combination with pioglitazone was investigated by measuring Glycosylated hemoglobin levels in mice. Male *db*/*db* (BKS.Cg- +*Lepr^{db}*/+*Lepr^{db}*) mice (6 weeks of age, CLEA Japan (Tokyo, Japan)) were divided into 4 groups (n=8 in each group) comprising Group A to Group D. Group A had free access to CE-2 powder chow (CLEA Japan) for 21 days. Group B had free access to CE-2 powder chow (CLEA Japan) containing 0.03% (w/w) of benzoate salt of Compound I for 21 days. The dose of Compound I in Group B was calculated to be 76.4±8.0 (mean ± SD) mg/kg body weight/day. Group C had free access to CE-2 powder chow (CLEA Japan) containing 0.0075% (w/w) of pioglitazone hydrochloride for 21 days. The dose of pioglitazone in Group C was calculated to be 15.4±1.5 (mean ± SD) mg/kg body weight/day. Group D had free access to CE-2 powder chow (CLEA Japan) containing 0.03% (w/w) of benzoate salt of Compound I in combination with 0.0075% (w/w) of pioglitazone hydrochloride for 21 days. The doses of Compound I and pioglitazone in Group D were calculated to be 56.5±3.1 (mean ± SD) mg/kg body weight/day and 14.1±0.8 (mean ± SD) mg/kg body weight/day, respectively. During 21 days of administration of the powder chow, there were not significant differences in the administration amount of the powder chow in the above 4 groups. After 21 days of administration of the powder chow, blood samples were taken from the orbital veins of the mice by capillary pipette under feeding condition, and Glycosylated hemoglobin levels were measured by HPLC-based method using TOSOH automated GHb Analyzer HLC-723 G7 (TOSOH, Japan).

The results are shown in Table 1. The values in the table means average (n=8) ± standard deviation.

**Table 1**

| **Group** | **Glycosylated Hemoglobin (%)** |
|---|---|
| Group A (Control) | 6.2 ± 0.4 |
| Group B (Compound I) | 5.8 ± 0.5 |
| Group C (Pioglitazone) | 5.0 ± 0.7 |
| Group D (Compound I + Pioglitazone) | 4.1 ± 0.6 |

As shown in Table 1, the combination of Compound I with pioglitazone showed excellent effects of lowering glycosylated hemoglobin levels.

### 8. EFFECT OF CO-ADMINISTRATION WITH VOGLIBOSE ON PLASMA GLUCOSE

The effect of administering Compound I in combination with voglibose was investigated by measuring plasma glucose levels in mice. Male *db*/*db* (BKS.Cg-+*Lepr^{db}*/+*Lepr^{db}*) mice (6 weeks of age, CLEA Japan (Tokyo, Japan)) were divided into 4 groups (n=6 in each group) comprising Group A to Group D. Group A had free access to CE-2 powder chow (CLEA Japan) for 21 days. Group B had free access to CE-2 powder chow (CLEA Japan) containing 0.03% (w/w) of benzoate salt of Compound I for 21 days. The dose of Compound I in Group B was calculated to be 72.8±1.8 (mean ± SD) mg/kg body weight/day. Group C had free access to CE-2 powder chow (CLEA Japan) containing 0.001%(w/w) of voglibose for 21 days. The dose of voglibose in Group C was calculated to be 1.8±0.1 (mean ± SD) mg/kg body weight/day. Group D had free access to CE-2 powder chow (CLEA Japan) containing 0.03% (w/w) of benzoate salt of Compound I in combination with 0.001% (w/w) of voglibose for 21 days. The doses of Compound I and voglibose in Group D were calculated to be 53.8±3.7 (mean ± SD) mg/kg body weight/day and 1.8±0.1 (mean ± SD) mg/kg body weight/day, respectively. During 21 days of administration of the powder chow, there were not significant differences in the administration amount of the powder chow in the above 4 groups. After 21 days of administration of the powder chow, blood samples were taken from the orbital veins of the mice by capillary pipette under feeding condition, and plasma glucose levels were enzymatically measured by using Autoanalyzer 7080 (Hitachi, Japan).

The results are shown in Table 2. The values in the table means average (n=6) ± standard deviation.

**Table 2**

| **Group** | **Plasma Glucose (mg/dL)** |
|---|---|
| Group A (control) | 398.7 ± 10.5 |
| Group D (Compound I + Voglibose) | 153.5 ± 18.5 |

As shown in Table 2, the combination of Compound I with voglibose showed excellent effects of lowering plasma glucose levels.
It will be apparent to those skilled in the art that various modifications and variations can be made to the compounds, compositions, kits, and methods of the present invention without departing from the spirit or scope of the invention. Thus, it is intended that the present invention cover the modifications and variations of this invention provided they come within the scope of the appended claims.

## Claims

1. A pharmaceutical composition formulated in a single dose form, wherein such single dose form comprises 12.5 mg of Compound I, wherein Compound I has the formula

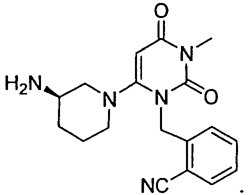

2. A pharmaceutical composition formulated in a single dose form, wherein such single dose form comprises 20 mg of Compound I, wherein Compound I has the formula

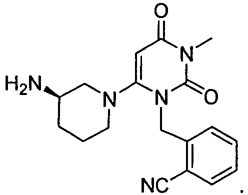

3. A pharmaceutical composition formulated in a single dose form, wherein such single dose form comprises 25mg of Compound I, wherein Compound I has the formula

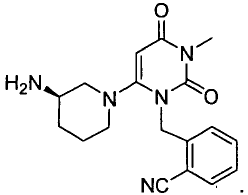

4. A pharmaceutical composition formulated in a single dose form, wherein such single dose form comprises 50 mg of Compound I, wherein Compound I has the formula

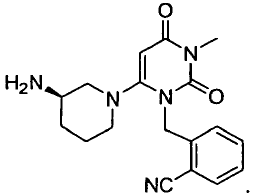

5. A pharmaceutical composition formulated in a single dose form, wherein such single dose form comprises 75 mg of Compound I, wherein Compound I has the formula

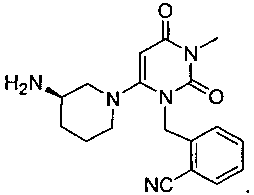

6. A pharmaceutical composition according to any one of claims 1-5, wherein such single dose form further comprises one or more antidiabetic compounds other than Compound I.

7. A pharmaceutical composition formulated in a single dose form wherein such single dose form comprises between 5 mg and 250 mg of Compound I and one or more antidiabetic compounds other than Compound I, wherein Compound I has the formula

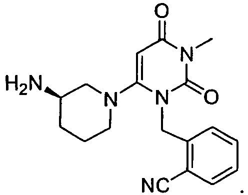

8. A pharmaceutical composition according to any one of claims 1-7, wherein such single dose form comprises one or more antidiabetic compounds selected from the group consisting of insulin signaling pathway modulators, compounds influencing a dysregulated hepatic glucose production, insulin sensitivity enhancers, and insulin secretion enhancers.

9. A pharmaceutical composition according to any one of claims 1-7, wherein such single dose form comprises one or more antidiabetic compounds selected from the group consisting of protein tyrosine phosphatase inhibitors, glutamine-fructose-6-phosphate amidotransferase inhibitors, glucose-6-phosphatase inhibitors, fructose-1,6-bisphosphatase inhibitors, glycogen phosphorylase inhibitors, glucagon receptor antagonists, phosphoenolpyruvate carboxykinase inhibitors, pyruvate dehydrogenase kinase inhibitors, alpha-glucosidase inhibitors, inhibitors of gastric emptying, insulin, and α₂-adrenergic antagonists.

10. A pharmaceutical composition according to any one of claims 1-7, wherein such single dose form comprises one or more antidiabetic compounds selected from the group consisting of GSK-3 inhibitors, retinoid X receptor agonists, Beta-3 AR agonists, UCP modulators, antidiabetic thiazolidinediones, non-glitazone type PPAR gamma agonists, dual PPAR gamma/PPAR alpha agonists, antidiabetic vanadium containing compounds and biguanides.

11. A pharmaceutical composition according to any one of claims 1-7, wherein such single dose form comprises one or more antidiabetic compounds selected from the group consisting of (S)-((3,4-dihydro-2-(phenyl-methyl)-2H-1-benzopyran-6-yl)methyl-thiazolidine-2,4-dione, 5-{[4-(3-(5-methyl-2-phenyl-4-oxazolyl)-1-oxo-propyl)-phenyl]-methyl}-thiazolidine-2,4-dione, 5-{[4-(1-methyl-cyclohexyl)methoxy)-phenyl]methyl]-thiazolidine-2,4-dione, 5-{[4-(2-(1-indolyl)ethoxy)phenyl]methyl}-thiazolidine-2,4-dione, 5-{4-[2-(5-methyl-2-phenyl-4-oxazoly)-ethoxy)]benzyl}-thiazolidine-2,4-dione, 5-(2-naphthylsulfonyl)-thiazolidine-2,4-dione, bis{4-[(2,4-dioxo-5-thiazolidinyl)-methyl]phenyl}methane, 5-{4-[2-(5-methyl-2-phenyl-4-oxazolyl)-2-hydroxyethoxy]-benzyl}- -thiazolidine-2,4-dione, 5-[4-(1-phenyl-1-cyclopropanecarbonylamino)-benzyl]-thiazolidine-2,4-dione, 5-{[4-(2-(2,3-dihydroindol-1-yl)ethoxy)phenylmethyl)-thiazolidine-2,4-dione, 5-[3-(4-chloro-phenyl])-2--propynyl]-5-phenylsulfonyl)thiazolidine-2,4-dione, 5-[3-(4-chlorophenyl])--2-propynyl]-5-(4-fluorophenyl-sulfonyl)thiazolidine-2,4-dione,5-{[4-(2-(methyl-2-pyridinyl-amino)-ethoxy)phenyl]methyl}-thiazolidine-2,4-dione, 5-([2-(2-naphthyl)-benzoxazol-5-yl]-methyl}thiazolidine-2,4-dione and 5-(2,4-dioxothiazolidin-5-ylmethyl)-2-methoxy-N-(4-trifluoromethyl-benzyl)benzamide, including any pharmaceutically acceptable salts thereof.

12. A pharmaceutical composition according to any one of claims 1-7, wherein such single dose form comprises metformin, including any pharmaceutically acceptable salts thereof.

13. A pharmaceutical composition according to any one of claims 1-7, wherein such single dose form comprises a sulphonyl urea derivative.

14. A pharmaceutical composition according to any one of claims 1-7, wherein such single dose form comprises one or more antidiabetic compounds selected from the group consisting of glisoxepid, glyburide, glibenclamide, acetohexamide, chloropropamide, glibornuride, tolbutamide, tolazamide, glipizide, carbutamide, gliquidone, glyhexamide, phenbutamide, tolcyclamide, glimepiride and gliclazide, including any pharmaceutically acceptable salts thereof.

15. A pharmaceutical composition according to any one of claims 1-7, wherein such single dose form comprises one or more antidiabetic compounds selected from the group consisting of incretin hormones or mimics thereof, beta-cell imidazoline receptor antagonists, and short-acting insulin secretagogues.

16. A pharmaceutical composition according to any one of claims 1-7, wherein such single dose form comprises insulin.

17. A pharmaceutical composition according to any one of claims 1-7, wherein such single dose form comprises one or more GLP-1 agonists.

18. A pharmaceutical composition according to any one of claims 1-7, wherein such single dose form comprises exenatide.

19. A pharmaceutical composition according to any one of claims 1-7, wherein such single dose form comprises one or more antidiabetic compounds selected from the group consisting of repaglinide, mitiglinide, and nateglinide, including any pharmaceutically acceptable salts thereof.

20. A pharmaceutical composition according to any one of claims 1-7, wherein such single dose form comprises one or more alpha-Glucosidase inhibitors.

21. A pharmaceutical composition according to any one of claims 1-7, wherein such single dose form comprises one or more antidiabetic compounds selected from the group consisting of acarbose, voglibose and miglitol, including any pharmaceutically acceptable salts thereof.

22. A pharmaceutical composition according to any one of claims 1-7, wherein such single dose form comprises rosiglitazone, including any pharmaceutically acceptable salts thereof.

23. A pharmaceutical composition according to any one of claims 1-7, wherein such single dose form comprises pioglitazone, including any pharmaceutically acceptable salts thereof.

24. A pharmaceutical composition according to any one of claims 1-7, wherein such single dose form comprises metformin and pioglitazone, including any pharmaceutically acceptable salts thereof.

25. A pharmaceutical composition according to any one of claims 23 and 24, wherein the pioglitazone comprises pioglitazone HCl.

26. A pharmaceutical composition according to any one of claims 1-25, wherein such single dose form is adapted for oral administration.

27. A pharmaceutical composition according to any one of claims 1-25, wherein such single dose form is a solid formulation adapted for oral administration.

28. A pharmaceutical composition according to any one of claims 1-25, wherein such single dose form is a tablet or capsule adapted for oral administration.

29. A pharmaceutical composition according to any one of claims 1-25, wherein such single dose form comprises an extended release formulation adapted for oral administration.

30. A pharmaceutical composition according to any one of claims 1-29, wherein Compound I is present in the pharmaceutical composition as a free base.

31. A pharmaceutical composition according to any one of claims 1-29, wherein Compound I is present in the pharmaceutical composition in a pharmaceutically acceptable salt.

32. A pharmaceutical composition according to any one of claims 1-29, wherein Compound I is present in the pharmaceutical composition in a benzoate salt.

33. A pharmaceutical composition according to any one of claims 1-29, wherein Compound I is present in the pharmaceutical composition in a toluenesulfonate salt.

34. A pharmaceutical composition according to any one of claims 1-29, wherein Compound I is present in the pharmaceutical composition in a hydrochloric acid salt.

35. A pharmaceutical composition formulated in a single dose form wherein such single dose form comprises between 5 mg and 250 mg of Compound I, wherein Compound I is present in the pharmaceutical composition in a benzoate salt and has the formula 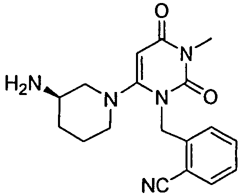 with the exception of the following compositions:
(a) a pharmaceutical composition formulated for oral administration consisting of 10 to 100 mg of Compound I, 105 mg of citric acid monohydrate, 18 mg of sodium hydroxide, a flavouring and water to 100 ml; and
(b) a pharmaceutical composition formulated for intravenous administration consisting of 5 to 10 mg of Compound I, 1.05 mg of citric acid monohydrate, 0.18 mg of sodium hydroxide, a sufficient quantity of dextrose monohydrate to make the formulation isotonic, and water to 1 ml.

36. A pharmaceutical composition formulated in a single dose form wherein such single dose form comprises between 5 mg and 250 mg of Compound I, wherein Compound I is present in the pharmaceutical composition in a toluenesulfonate salt and has the formula

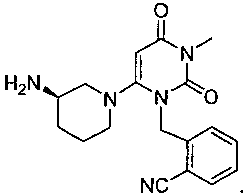

37. A pharmaceutical composition formulated in a single dose form wherein such single dose form comprises between 5 mg and 250 mg of Compound I, wherein Compound I is present in the pharmaceutical composition in a hydrochloric acid salt and has the formula

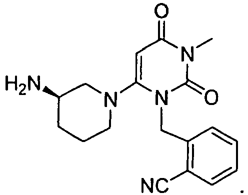

38. A kit comprising:
multiple doses of a pharmaceutical composition according to any one of claims 1-34, 36 or 37; and
instructions which comprise one or more forms of information selected from the group consisting of indicating a disease state for which the pharmaceutical composition is to be administered, storage information for the pharmaceutical composition, dosing information and instructions regarding how to administer the pharmaceutical composition.

39. An article of manufacture comprising:
multiple doses of a pharmaceutical composition according to any one of claims 1-34, 36 or 37; and
packaging materials.

40. An article of manufacture according to claim 39, wherein the packaging material comprises a container for housing the multiple doses of the pharmaceutical composition.

41. An article of manufacture according to claim 40, wherein the container comprises a label indicating one or more members of the group consisting of a disease state for which the compound is to be administered, storage information, dosing information and/or instructions regarding how to administer the composition.

42. Use of a pharmaceutical composition according to any one of claims 1-34, 36 or 37 in combination with one or more antidiabetic compounds other than Compound I for the manufacture of a pharmaceutical for treating Type II diabetes, wherein Compound I has the formula

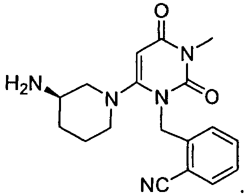

43. Use of a pharmaceutical composition according to any one of claims 1-34, 36 or 37 for the manufacture of a pharmaceutical comprising a combination of Compound I and one or more antidiabetic compounds other than Compound I for treating Type II diabetes, wherein Compound I has the formula

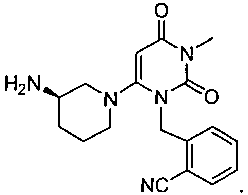

44. Use of one or more antidiabetic compounds other than Compound I for the manufacture of a pharmaceutical comprising a combination of a pharmaceutical composition according to any one of claims 1-34, 36 or 37 and one or more antidiabetic compounds other than Compound I for treating Type II diabetes, wherein Compound I has the formula

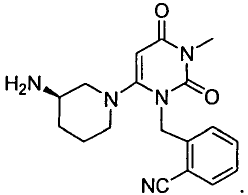

45. Use of a pharmaceutical composition according to any one of claims 1-34, 36 or 37 in combination with one or more antidiabetic compounds other than Compound I for the manufacture of a pharmaceutical for treating Type I diabetes, wherein Compound I has the formula

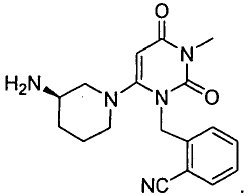

46. Use of a pharmaceutical composition according to any one of claims 1-34, 36 or 37 for the manufacture of a pharmaceutical comprising a combination of Compound I and one or more antidiabetic compounds other than Compound I for treating Type I diabetes, wherein Compound I has the formula

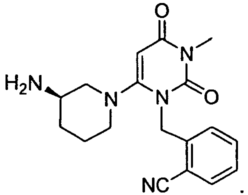

47. Use of one or more antidiabetic compounds other than Compound I for the manufacture of a pharmaceutical comprising a combination of a pharmaceutical composition according to any one of claims 1-34, 36 or 37 and one or more antidiabetic compounds other than Compound I for treating Type I diabetes, wherein Compound I has the formula

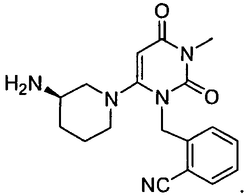

## Patentansprüche

1. Pharmazeutische Zusammensetzung, die in einer Einzeldosisform formuliert ist, wobei eine derartige Einzeldosisform 12,5 mg Verbindung I umfasst, wobei Verbindung I die Formel 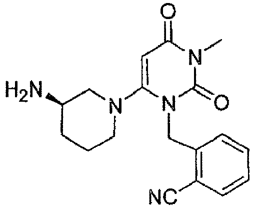 aufweist.

2. Pharmazeutische Zusammensetzung, die in einer Einzeldosisform formuliert ist, wobei eine derartige Einzeldosisform 20 mg Verbindung I umfasst, wobei Verbindung I die Formel 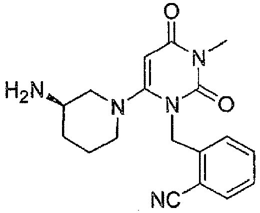 aufweist.

3. Pharmazeutische Zusammensetzung, die in einer Einzeldosisform formuliert ist, wobei eine derartige Einzeldosisform 25 mg Verbindung I umfasst, wobei Verbindung I die Formel 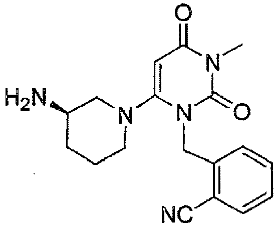 aufweist.

4. Pharmazeutische Zusammensetzung, die in einer Einzeldosisform formuliert ist, wobei eine derartige Einzeldosisform 50 mg Verbindung I umfasst, wobei Verbindung I die Formel 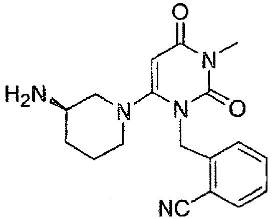 aufweist.

5. Pharmazeutische Zusammensetzung, die in einer Einzeldosisform formuliert ist, wobei eine derartige Einzeldosisform 75 mg Verbindung I umfasst, wobei Verbindung I die Formel 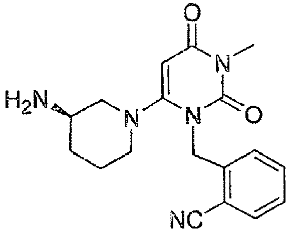 aufweist.

6. Pharmazeutische Zusammensetzung nach einem der Ansprüche 1-5, wobei eine derartige Einzeldosisform eine oder mehrere antidiabetische Verbindungen, die von Verbindung I verschieden sind, umfasst.

7. Pharmazeutische Zusammensetzung, die in einer Einzeldosisform formuliert ist, wobei eine derartige Einzeldosisform zwischen 5 mg und 250 mg Verbindung I und eine oder mehrere antidiabetische Verbindungen, die von Verbindung I verschieden sind, umfasst, wobei Verbindung I die Formel 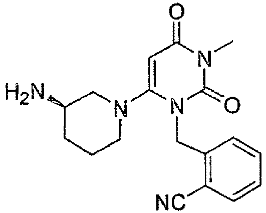 aufweist.

8. Pharmazeutische Zusammensetzung nach einem der Ansprüche 1-7, wobei eine derartige Einzeldosisform eine oder mehrere antidiabetische Verbindungen aus der Gruppe bestehend aus Insulinsignalweg-Modulatoren, Verbindungen, die eine fehlregulierte hepatische Glucoseproduktion beeinflussen, Insulinempfindlichkeitsverstärkern und Insulinausscheidungsverstärkern umfasst.

9. Pharmazeutische Zusammensetzung nach einem der Ansprüche 1-7, wobei eine derartige Einzeldosisform eine oder mehrere antidiabetische Verbindungen aus der Gruppe bestehend aus Protein-Tyrosinphosphatase-Inhibitoren, Glutamin-Fructose-6-Phosphat-Amidotransferase-Inhibitoren, Glucose-6-Phosphatase-Inhibitoren, Fructose-1,6-Biphosphatase-Inhibitoren, Glykogen-Phosphorylase-Inhibitoren, Glucagon-Rezeptorantagonisten, Phosphoenolpyruvat-Carboxykinase-Inhibitoren, Pyruvat-Dehydrogenase-Kinase-Inhibitoren, Alpha-Glucosidase-Inhibitoren, Inhibitoren der Magenentleerung, Insulin und α₂-adrenergen Antagonisten umfasst.

10. Pharmazeutische Zusammensetzung nach einem der Ansprüche 1-7, wobei eine derartige Einzeldosisform eine oder mehrere antidiabetische Verbindungen aus der Gruppe bestehend aus GSK-3-Inhibitoren, Retinoid-X-Rezeptor-Agonisten, Beta-3-AR-Agonisten, UCP-Modulatoren, antidiabetischen Thiazolidindionen, PPAR-Gamma-Agonisten vom Nicht-Glitazon-Typ, dualen PPAR-Gamma/PPAR-Alpha-Agonisten, antidiabetischen Vanadium enthaltenden Verbindungen und Biguaniden umfasst.

11. Pharmazeutische Zusammensetzung nach einem der Ansprüche 1-7, wobei eine derartige Einzeldosisform eine oder mehrere antidiabetische Verbindungen aus der Gruppe bestehend aus (S)-((3,4-Dihydro-2-(phenyl-methyl)-2H-1-benzopyran-6-yl)-methyl-thiazolidin-2,4-dion, 5-{[4-(3-(5-Methyl-2-phenyl-4-oxazolyl)-1-oxo-propyl)-phenyl]-methyl}-thiazolidin-2,4-dion, 5-{[4-(1-Methyl-cyclohexyl)-methoxy)-phenyl]methyl]-thiazolidin-2,4-dion, 5-{[4-(2-(1-Indolyl)ethoxy)phenyl]methyl}-thiazoli-din-2,4-dion, 5-{4-[2-(5-Methyl-2-phenyl-4-oxazolyl)-ethoxy)]benzyl}-thiazolidin-2,4-dion, 5-(2-Naphthylsulfonyl)-thiazolidin-2,4-dion, Bis{4-[(2,4-dioxo-5-thiazolidinyl)-methyl]phenyl}methan, 5-{4-[2-(5-Methyl-2-phenyl-4-oxazolyl)-2-hydroxy-ethoxy]-benzyl}-thiazolidin-2,4-dion, 5-[4-(1-Phenyl-1-cyclopropancarbonylamino)-benzyl]-thiazolidin-2,4-dion, 5-{[4-(2-(2,3-Dihydroindol-1-yl)ethoxy)phenylmethyl)-thiazolidin-2,4-dion, 5-[3-(4-Chlorphenyl])-2-propinyl]-5-phenylsulfonyl)-thiazolidin-2,4-dion, 5-[3-(4-Chlcrphényl])-2-propinyl]-5-(4-fluorphenyl-sulfonyl)thiazolidin-2,4-dion, 5-{[4-(2-(Methyl-2-pyridinyl-amino)-ethoxy)phenyl]methyl}-thiazolidin-2,4-dion, 5-([2-(2-Naphthyl)-benzoxazol-5-yl]-methyl}thiazolidin-2,4-dion und 5-(2,4-Dioxothiazolidin-5-ylmethyl)-2-methoxy-N-(4-trifluormethyl-benzyl)benzamid einschließlich pharmazeutisch unbedenklicher Salze davon umfasst.

12. Pharmazeutische Zusammensetzung nach einem der Ansprüche 1-7, wobei eine derartige Einzeldosisform Metformin einschließlich pharmazeutisch unbedenklicher Salze davon umfasst.

13. Pharmazeutische Zusammensetzung nach einem der Ansprüche 1-7, wobei eine derartige Einzeldosisform ein Sulfonylharnstoff-Derivat umfasst.

14. Pharmazeutische Zusammensetzung nach einem der Ansprüche 1-7, wobei eine derartige Einzeldosisform eine oder mehrere antidiabetische Verbindungen aus der Gruppe bestehend aus Glisoxepid, Glyburid, Glibenclamid, Acetohexamid, Chloropropamid, Glibornurid, Tolbutamid, Tolazamid, Glipizid, Carbutamid, Gliquidon, Glyhexamid, Phenbutamid, Tolcyclamid, Glimepirid und Gliclazid einschließlich pharmazeutisch unbedenklicher Salze davon umfasst.

15. Pharmazeutische Zusammensetzung nach einem der Ansprüche 1-7, wobei eine derartige Einzeldosisform eine oder mehrere antidiabetische Verbindungen aus der Gruppe bestehend aus Inkretinhormonen oder Nachahmern davon, Betazellen-Imidazolin-Rezeptorantagonisten und kurzwirkenden Insulinsekretagoga umfasst.

16. Pharmazeutische Zusammensetzung nach einem der Ansprüche 1-7, wobei eine derartige Einzeldosisform Insulin umfasst.

17. Pharmazeutische Zusammensetzung nach einem der Ansprüche 1-7, wobei eine derartige Einzeldosisform einen oder mehrere GLP-1-Agonisten umfasst.

18. Pharmazeutische Zusammensetzung nach einem der Ansprüche 1-7, wobei eine derartige Einzeldosisform Exenatid umfasst.

19. Pharmazeutische Zusammensetzung nach einem der Ansprüche 1-7, wobei eine derartige Einzeldosisform eine oder mehrere antidiabetische Verbindungen aus der Gruppe bestehend aus Repaglinid, Mitiglinid und Nateglinid einschließlich pharmazeutisch unbedenklicher Salze davon umfasst.

20. Pharmazeutische Zusammensetzung nach einem der Ansprüche 1-7, wobei eine derartige Einzeldosisform einen oder mehrere Alpha-Glucosidase-Inhibitoren umfasst.

21. Pharmazeutische Zusammensetzung nach einem der Ansprüche 1-7, wobei eine derartige Einzeldosisform eine oder mehrere antidiabetische Verbindungen aus der Gruppe bestehend aus Acarbose, Voglibose und Miglitol einschließlich pharmazeutisch unbedenklicher Salze davon umfasst.

22. Pharmazeutische Zusammensetzung nach einem der Ansprüche 1-7, wobei eine derartige Einzeldosisform Rosiglitazon einschließlich pharmazeutisch unbedenklicher Salze davon umfasst.

23. Pharmazeutische Zusammensetzung nach einem der Ansprüche 1-7, wobei eine derartige Einzeldosisform Pioglitazon einschließlich pharmazeutisch unbedenklicher Salze davon umfasst.

24. Pharmazeutische Zusammensetzung nach einem der Ansprüche 1-7, wobei eine derartige Einzeldosisform Metformin und Pioglitazon einschließlich pharmazeutisch unbedenklicher Salze davon umfasst.

25. Pharmazeutische Zusammensetzung nach einem der Ansprüche 23 und 24, wobei das Pioglitazon Pioglitazon-HCl umfasst.

26. Pharmazeutische Zusammensetzung nach einem der Ansprüche 1-25, wobei eine derartige Einzeldosisform für die orale Verabreichung geeignet ist.

27. Pharmazeutische Zusammensetzung nach einem der Ansprüche 1-25, wobei es sich bei einer derartigen Einzeldosisform um eine feste Formulierung, die für die orale Verabreichung geeignet ist, handelt.

28. Pharmazeutische Zusammensetzung nach einem der Ansprüche 1-25, wobei es sich bei einer derartigen Einzeldosisform um eine Tablette oder Kapsel, die für die orale Verabreichung geeignet ist, handelt.

29. Pharmazeutische Zusammensetzung nach einem der Ansprüche 1-25, wobei eine derartige Einzeldosisform eine Retardformulierung, die für die orale Verabreichung geeignet ist, umfasst.

30. Pharmazeutische Zusammensetzung nach einem der Ansprüche 1-29, wobei Verbindung I in der pharmazeutischen Zusammensetzung als freie Base vorliegt.

31. Pharmazeutische Zusammensetzung nach einem der Ansprüche 1-29, wobei Verbindung I in der pharmazeutischen Zusammensetzung in einem pharmazeutisch unbedenklichen Salz vorliegt.

32. Pharmazeutische Zusammensetzung nach einem der Ansprüche 1-29, wobei Verbindung I in der pharmazeutischen Zusammensetzung in einem Benzoatsalz vorliegt.

33. Pharmazeutische Zusammensetzung nach einem der Ansprüche 1-29, wobei Verbindung I in der pharmazeutischen Zusammensetzung in einem Toluolsulfonatsalz vorliegt.

34. Pharmazeutische Zusammensetzung nach einem der Ansprüche 1-29, wobei Verbindung I in der pharmazeutischen Zusammensetzung in einem Salzsäuresalz vorliegt.

35. Pharmazeutische Zusammensetzung, die in einer Einzeldosisform formuliert ist, wobei eine derartige Einzeldosisform zwischen 5 mg und 250 mg Verbindung I umfasst, wobei Verbindung I in der pharmazeutischen Zusammensetzung in einem Benzoatsalz vorliegt und die Formel 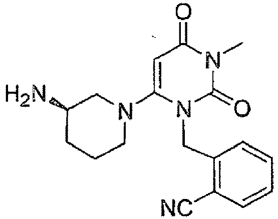 aufweist,
mit Ausnahme der folgenden Zusammensetzungen:
(a) einer zur oralen Verabreichung formulierten pharmazeutischen Zusammensetzung, die 10 bis 100 mg Verbindung I, 105 mg Citronensäuremonohydrat, 18 mg Natriumhydroxid, einen Geschmacksstoff und Wasser auf 100 ml umfasst; und
(b) einer zur intravenösen Verabreichung formulierten pharmazeutischen Zusammensetzung, die 5 bis 10 mg Verbindung I, 1,05 mg Citronensäuremonohydrat, 0,18 mg Natriumhydroxid, eine zum Isotonischmachen der Formulierung ausreichende Menge Dextrosemonohydrat und Wasser auf 1 ml umfasst.

36. Pharmazeutische Zusammensetzung, die in einer Einzeldosisform formuliert ist, wobei eine derartige Einzeldosisform zwischen 5 mg und 250 mg Verbindung I umfasst, wobei Verbindung I in der pharmazeutischen Zusammensetzung in einem Toluolsulfonatsalz vorliegt und die Formel 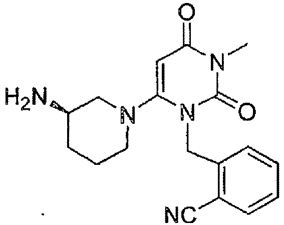 aufweist.

37. Pharmazeutische Zusammensetzung, die in einer Einzeldosisform formuliert ist, wobei eine derartige Einzeldosisform zwischen 5 mg und 250 mg Verbindung I umfasst, wobei Verbindung I in der pharmazeutischen Zusammensetzung in einem Salzsäuresalz vorliegt und die Formel 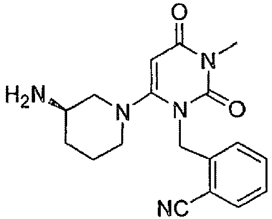 aufweist.

38. Kit, umfassend:
mehrere Dosen einer pharmazeutischen Zusammensetzung nach einem der Ansprüche 1-34, 36 oder 37 und
Anweisungen, die eine oder mehrere Informationen aus der Gruppe bestehend aus der Angabe eines Krankheitszustands, für den die pharmazeutische Zusammensetzung zu verabreichen ist, Aufbewahrungsinformationen für die pharmazeutische Zusammensetzung, Dosierungsinformationen und Anweisungen zur Verabreichung der pharmazeutischen Zusammensetzung umfassen.

39. Erzeugnis, umfassend:
mehrere Dosen einer pharmazeutischen Zusammensetzung nach einem der Ansprüche 1-34, 36 oder 37 und
Verpackungsmaterialien.

40. Erzeugnis nach Anspruch 39, wobei das Verpackungsmaterial einen Behälter zur Aufnahme der mehreren Dosen der pharmazeutischen Zusammensetzung umfasst.

41. Erzeugnis nach Anspruch 40, wobei der Behälter ein Etikett umfasst, auf dem ein oder mehrere Elemente der Gruppe bestehend aus einem Erkrankungszustand, für den die Zusammensetzung verabreicht werden soll, Aufbewahrungsinformationen, Dosierungsinformationen und/oder Anweisungen zur Verabreichung der Zusammensetzung angegeben sind.

42. Verwendung einer pharmazeutischen Zusammensetzung nach einem der Ansprüche 1-34, 36 oder 37 in Kombination mit einer oder mehreren antidiabetischen Verbindungen, die von Verbindung I verschieden sind, zur Herstellung eines Pharmazeutikums zur Behandlung von Typ-II-Diabetes, wobei die Verbindung I die Formel 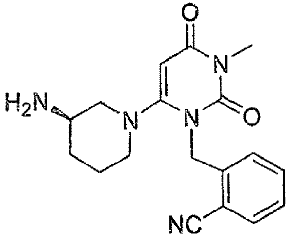 aufweist.

43. Verwendung einer pharmazeutischen Zusammensetzung nach einem der Ansprüche 1-34, 36 oder 37 zur Herstellung eines Pharmazeutikums, das eine Kombination von Verbindung I und einer oder mehreren antidiabetischen Verbindungen, die von Verbindung I verschieden sind, umfasst, zur Behandlung von Typ-II-Diabetes, wobei die Verbindung I die Formel 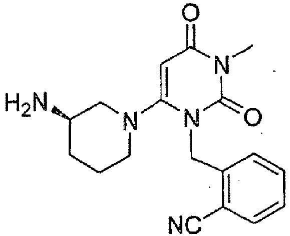 aufweist.

44. Verwendung einer oder mehrerer antidiabetischer Verbindungen, die von Verbindung I verschieden sind, zur Herstellung eines Pharmazeutikums, das eine Kombination einer pharmazeutischen Zusammensetzung nach einem der Ansprüche 1-34, 36 oder 37 und einer oder mehrerer antidiabetischer Verbindungen, die von Verbindung I verschieden sind, umfasst, zur Behandlung von Typ-II-Diabetes, wobei die Verbindung I die Formel 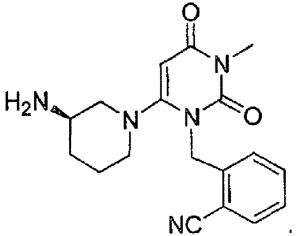 aufweist.

45. Verwendung einer pharmazeutischen Zusammensetzung nach einem der Ansprüche 1-34, 36 oder 37 in Kombination mit einer oder mehreren antidiabetischen Verbindungen, die von Verbindung I verschieden sind, zur Herstellung eines Pharmazeutikums zur Behandlung von Typ-I-Diabetes, wobei die Verbindung I die Formel 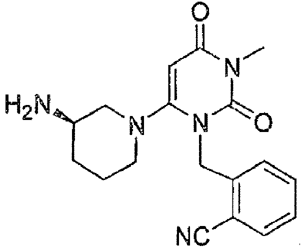 aufweist.

46. Verwendung einer pharmazeutischen Zusammensetzung nach einem der Ansprüche 1-34, 36 oder 37 zur Herstellung eines Pharmazeutikums, das eine Kombination von Verbindung I und einer oder mehreren antidiabetischen Verbindungen, die von Verbindung I verschieden sind, umfasst, zur Behandlung von Typ-I-Diabetes, wobei die Verbindung I die Formel 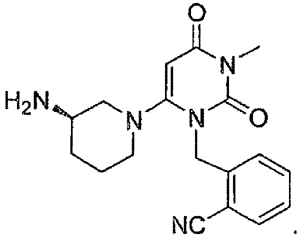 aufweist.

47. Verwendung einer oder mehrerer antidiabetischer Verbindungen, die von Verbindung I verschieden sind, zur Herstellung eines Pharmazeutikums, das eine Kombination einer pharmazeutischen Zusammensetzung nach einem der Ansprüche 1-34, 36 oder 37 und einer oder mehrerer antidiabetischer Verbindungen, die von Verbindung I verschieden sind, umfasst, zur Behandlung von Typ-I-Diabetes, wobei die Verbindung I die Formel 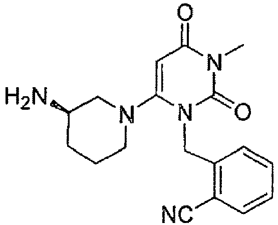 aufweist.

## Revendications

1. Composition pharmaceutique formulée en une forme galénique unitaire, où une telle forme galénique unitaire comprend entre 12,5 mg de Composé I, le Composé I répondant à la formule

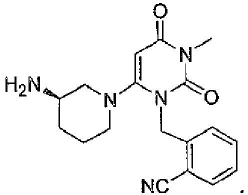

2. Composition pharmaceutique formulée en une forme galénique unitaire, où une telle forme galénique unitaire comprend 20 mg de Composé I, le Composé I répondant à la formule

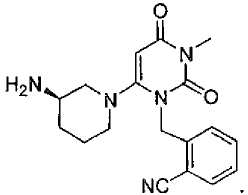

3. Composition pharmaceutique formulée en une forme galénique unitaire, où une telle forme galénique unitaire comprend 25 mg de Composé I, le Composé I répondant à la formule

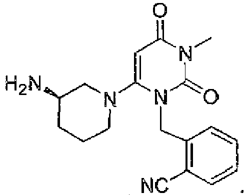

4. Composition pharmaceutique formulée en une forme galénique unitaire, où une telle forme galénique unitaire comprend 50 mg de Composé I, le Composé I répondant à la formule

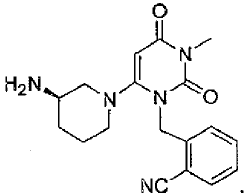

5. Composition pharmaceutique formulée en une forme galénique unitaire, où une telle forme galénique unitaire comprend 75 mg de Composé I, le Composé I répondant à la formule

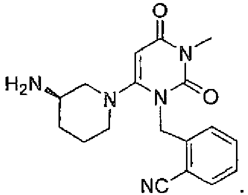

6. Composition pharmaceutique conforme à l'une quelconque des revendications 1 à 5, où une telle forme galénique unitaire comprend en outre un ou plusieurs composés antidiabétiques différents du Composé I.

7. Composition pharmaceutique formulée en une forme galénique unitaire, où une telle forme galénique unitaire comprend entre 5 mg et 250 mg de Composé I et un ou plusieurs composés antidiabétiques différents du Composé I, le Composé I répondant à la formule

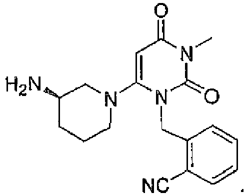

8. Composition pharmaceutique conforme à l'une quelconque des revendications 1 à 7, où une telle forme galénique unitaire comprend un ou plusieurs composés antidiabétiques choisis dans le groupe constitué par les modulateurs de la voie de signalement de l'insuline, les composés entraînant une dérégulation de la production hépatique de glucose, les agents amplifiant la sensibilité à l'insuline et les agents amplifiant la sécrétion d'insuline.

9. Composition pharmaceutique conforme à l'une quelconque des revendications 1 à 7, où ladite forme galénique unitaire comprend un ou plusieurs composés antidiabétiques choisis dans le groupe constitué par les inhibiteurs de protéine tyrosine phosphatase, les inhibiteurs de glutamine-fructose-6-phosphate amidotransférase, les inhibiteurs de glucose-6-phosphatase, les inhibiteurs de fructose-1,6-bisphosphatase, les inhibiteurs de glycogène phosphorylase, les antagonistes de récepteurs de glucagon, les inhibiteurs de phosphénolpyruvate carboxykinase, les inhibiteurs de pyruvate déshydrogénase kinase, les inhibiteurs d'alpha-glucosidase, les inhibiteurs de gastroparésie, l'insuline et les antagonistes α₂-adrénérgiques.

10. Composition pharmaceutique conforme à l'une quelconque des revendications 1 à 7, où une telle forme galénique unitaire comprend un ou plusieurs composés antidiabétiques choisis dans le groupe constitué par les inhibiteurs de GSK-3, les agonistes de récepteur X des rétinoïdes, les agonistes adrénergiques Bêta-3, les modulateurs d'UCP, les thiazolidinediones antidiabétiques, les agonistes de PPAR gamma de type non-glitazone, les agonistes doubles PPAR gamma/PPAR alpha, les composés antidiabétiques comportant du vanadium et les biguanides.

11. Composition pharmaceutique conforme à l'une quelconque des revendications 1 à 7, où une telle forme galénique unitaire comprend un ou plusieurs composés antidiabétiques choisis dans le groupe constitué par les suivants :
(S)-((3,4-dihydro-2-(phényl-méthyl)-2H-1-benzopyran-6-yl)méthyl-thiazolidine-2,4-dione,
5-{[4-(3-(5-méthyl-2-phényl-4-oxazolyl)-1-oxo-propyl)-phényl]-méthyl}-thiazolidine-2,4-dione,
5-{[4-(1-méthyl-cyclohéxyl)méthoxy)-phényl]méthyl]-thiazolidine-2,4-dione,
5-{[4-(2-(1-indolyl)éthoxy)phényl]méthyl}-thiazolidine-2,4-dione,
5-{4-[2-(5-méthyl-2-phényl-4-oxazoly)-éthoxy) ]benzyl}-thiazolidine-2,4-dione,
5-(2-naphtylsulfonyl)-thiazolidine-2,4-dione, bis{4-[(2,4-dioxo-5-thiazolidinyl)-méthyl]phényl}méthane,
5-{4-[2-(5-méthyl-2-phényl-4-oxazolyl)-2-hydroxyéthoxy]-benzyl}--thiazolidine-2,4-dione,
5-[4-(1-phényl-1-cyclopropanecarbonylamino)-benzyl]-thiazolidine-2,4-dione,
5-{[4-(2-(2,3-dihydroindol-1-yl)éthoxy)phénylméthyl)-thiazolidine-2,4-dione,
5-[3-(4-chloro-phényl])-2--propynyl]-5-phénylsulfonyl)thiazolidine-2,4-dione,
5-[3-(4-chlorophényl])--2-propynyl]-5-(4-fluorophényl-sulfonyl)thiazolidine-2,4-dione,
5-{[4-(2-(méthyl-2-pyridinyl-amino)-éthoxy)phényl]méthyl}-thiazolidine-2,4-dione,
5-([2-(2-naphtyl)-benzoxazol-5-yl]-méthyl}thiazolidine-2,4-dione et 5-(2,4-dioxothiazolidin-5-ylméthyl)-2-méthoxy-N-(4-trifluorométhyl-benzyl)benzamide,
y compris leurs éventuels sels de qualité pharmaceutique.

12. Composition pharmaceutique conforme à l'une quelconque des revendications 1 à 7, où une telle forme galénique unitaire comprend de la metformine, y compris ses éventuels sels de qualité pharmaceutique.

13. Composition pharmaceutique conforme à l'une quelconque des revendications 1 à 7, où une telle forme galénique unitaire comprend un dérivé de sulfonylurée.

14. Composition pharmaceutique conforme à l'une quelconque des revendications 1 à 7, où une telle forme galénique unitaire comprend un ou plusieurs composés antidiabétiques choisis dans le groupe constitué par les suivants : glisoxépide, glyburide, glibenclamide, acétohexamide, chloropropamide, glibornuride, tolbutamide, tolazamide, glipizide, carbutamide, gliquidone, glyhexamide, phenbutamide, tolcyclamide, glimépiride et gliclazide, y compris leurs éventuels sels de qualité pharmaceutique.

15. Composition pharmaceutique conforme à l'une quelconque des revendications 1 à 7, où une telle forme galénique unitaire comprend un ou plusieurs composés antidiabétiques choisis dans le groupe constitué par les hormones de type incrétine ou leurs mimétiques, les antagonistes de récepteurs d'imidazoline et de cellules bêta, et les sécrétagogues de l'insuline à courte durée d'action.

16. Composition pharmaceutique conforme à l'une quelconque des revendications 1 à 7, où une telle forme galénique unitaire comprend de l'insuline.

17. Composition pharmaceutique conforme à l'une quelconque des revendications 1 à 7, où une telle forme galénique unitaire comprend un ou plusieurs agonistes de GLP-1.

18. Composition pharmaceutique conforme à l'une quelconque des revendications 1 à 7, où une telle forme galénique unitaire comprend de l'exénatide.

19. Composition pharmaceutique conforme à l'une quelconque des revendications 1 à 7, où une telle forme galénique unitaire comprend un ou plusieurs composés antidiabétiques choisis dans le groupe constitué par les suivants : répaglinide, mitiglinide et natéglinide, y compris leurs éventuels sels de qualité pharmaceutique.

20. Composition pharmaceutique conforme à l'une quelconque des revendications 1 à 7, où une telle forme galénique unitaire comprend un ou plusieurs agonistes d'alpha-glucosidase.

21. Composition pharmaceutique conforme à l'une quelconque des revendications 1 à 7, où une telle forme galénique unitaire comprend un ou plusieurs composés antidiabétiques choisis dans le groupe constitué par les suivants : acarbose, voglibose et miglitol, y compris leurs éventuels sels de qualité pharmaceutique.

22. Composition pharmaceutique conforme à l'une quelconque des revendications 1 à 7, où une telle forme galénique unitaire comprend de la rosiglitazone, y compris ses éventuels sels de qualité pharmaceutique.

23. Composition pharmaceutique conforme à l'une quelconque des revendications 1 à 7, où une telle forme galénique unitaire comprend de la pioglitazone, y compris ses éventuels sels de qualité pharmaceutique.

24. Composition pharmaceutique conforme à l'une quelconque des revendications 1 à 7, où une telle forme galénique unitaire comprend de la metformine et de la pioglitazone, y compris leurs éventuels sels de qualité pharmaceutique.

25. Composition pharmaceutique conforme à l'une quelconque des revendications 23 et 24, où le pioglitazone comprend le pioglitazone HCl.

26. Composition pharmaceutique conforme à l'une quelconque des revendications 1 à 25, où une telle forme galénique unitaire est adaptée à une administration orale.

27. Composition pharmaceutique conforme à l'une quelconque des revendications 1 à 25, où une telle forme galénique unitaire est une formule solide adaptée à une administration orale.

28. Composition pharmaceutique conforme à l'une quelconque des revendications 1 à 25, où une telle forme galénique unitaire est un comprimé ou une capsule adapté(e) à une administration orale.

29. Composition pharmaceutique conforme à l'une quelconque des revendications 1 à 25, où une telle forme galénique unitaire comprend une formule à libération prolongée adaptée à une administration orale.

30. Composition pharmaceutique conforme à l'une quelconque des revendications 1 à 29, où le Composé I est présent dans la composition pharmaceutique sous forme d'une base libre.

31. Composition pharmaceutique conforme à l'une quelconque des revendications 1 à 29, où le Composé I est présent dans la composition pharmaceutique sous forme d'un sel de qualité pharmaceutique.

32. Composition pharmaceutique conforme à l'une quelconque des revendications 1 à 29, où le Composé I est présent dans la composition pharmaceutique sous forme d'un sel de benzoate.

33. Composition pharmaceutique conforme à l'une quelconque des revendications 1 à 29, où le Composé I est présent dans la composition pharmaceutique sous forme d'un sel de toluènesulfonate.

34. Composition pharmaceutique conforme à l'une quelconque des revendications 1 à 29, où le Composé I est présent dans la composition pharmaceutique sous forme d'un sel d'acide chlorhydrique.

35. Composition pharmaceutique formulée en une forme galénique unitaire, où une telle forme galénique unitaire comprend entre 5 mg et 250 mg de Composé I, le Composé I étant présent dans la composition pharmaceutique sous forme d'un sel de benzoate et répondant à la formule 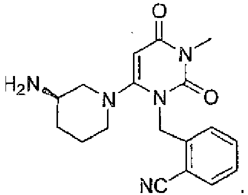 à l'exception des compositions suivantes :
(a) une composition pharmaceutique formulée pour administration orale comprenant entre 10 et 100 mg de Composé I, 105 mg de monohydrate d'acide citrique, 18 mg d'hydroxyde de sodium, un arôme et de l'eau pour compléter à 100 ml ; et
(b) une composition pharmaceutique formulée pour administration intraveineuse comprenant entre 5 et 10 mg de Composé I, 1,05 mg de monohydrate d'acide citrique, 0,18 mg d'hydroxyde de sodium, une quantité suffisante de monohydrate de dextrose pour rendre la formule isotonique, et de l'eau pour compléter à 1 ml.

36. Composition pharmaceutique formulée en une forme galénique unitaire, où une telle forme galénique unitaire comprend entre 5 mg et 250 mg de Composé I, le Composé I étant présent dans la composition pharmaceutique sous forme d'un sel de toluènesulfonate et répondant à la formule

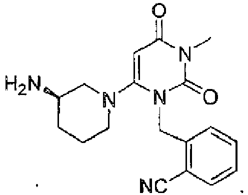

37. Composition pharmaceutique formulée en une forme galénique unitaire, où une telle forme galénique unitaire comprend entre 5 mg et 250 mg de Composé I, le Composé I étant présent dans la composition pharmaceutique sous forme d'un sel d'acide chlorhydrique et répondant à la formule

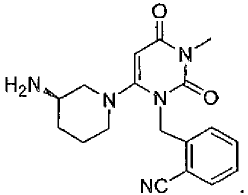

38. Trousse comprenant :
plusieurs doses d'une composition pharmaceutique conforme à l'une quelconque des revendications 1 à 34 ; et
des instructions qui comprennent une ou plusieurs formes d'informations choisies dans le groupe constitué par l'indication d'un état pathologique pour lequel la composition pharmaceutique doit être administrée, des informations de conservation pour la composition pharmaceutique, des informations posologiques et des instructions relatives à l'administration de la composition pharmaceutique.

39. Article manufacturé comprenant :
plusieurs doses d'une composition pharmaceutique conforme à l'une quelconque des revendications 1 à 34 ; et
des matériels d'emballage.

40. Article manufacturé conforme à la revendication 39, où le matériel d'emballage comprend un récipient destiné à accueillir les multiples doses de la composition pharmaceutique.

41. Article manufacturé conforme à la revendication 40, où le récipient comprend une étiquette indiquant un ou plusieurs membres du groupe constitué par un état pathologique pour lequel le composé doit être administré, des informations de conservation, des informations posologiques et/ou des instructions relatives à l'administration de la composition.

42. Emploi d'une composition pharmaceutique conforme à l'une quelconque des revendications 1 à 34, 36 et 37 en combinaison avec un ou plusieurs composés antidiabétiques différents du Composé I dans la fabrication d'un produit pharmaceutique destiné au traitement du diabète de type II, où le Composé I répond à la formule

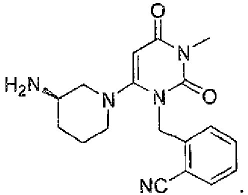

43. Emploi d'une composition pharmaceutique conforme à l'une quelconque des revendications 1 à 34, 36 et 37 dans la fabrication d'un produit pharmaceutique comprenant une combinaison du Composé I et d'un ou de plusieurs composés antidiabétiques différents du Composé I, destiné au traitement du diabète de type II, où le Composé I répond à la formule

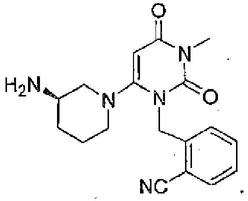

44. Emploi d'un ou de plusieurs composés antidiabétiques différents du Composé I dans la fabrication d'un produit pharmaceutique comprenant une combinaison d'une composition pharmaceutique conforme à l'une quelconque des revendications 1 à 34, 36 et 37 et d'un ou de plusieurs composés antidiabétiques différents du Composé I, destiné au traitement du diabète de type II, où le Composé I répond à la formule

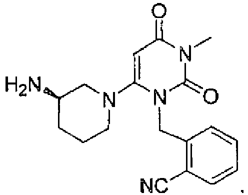

45. Emploi d'une composition pharmaceutique conforme à l'une quelconque des revendications 1 à 34, 36 et 37 en combinaison avec un ou plusieurs composés antidiabétiques différents du Composé I dans la fabrication d'un produit pharmaceutique destiné au traitement du diabète de type I, où le Composé I répond à la formule

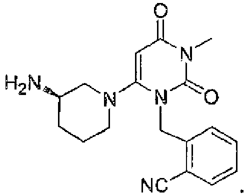

46. Emploi d'une composition pharmaceutique conforme à l'une quelconque des revendications 1 à 34, 36 et 37 dans la fabrication d'un produit pharmaceutique comprenant une combinaison du Composé I et d'un ou de plusieurs composés antidiabétiques différents du Composé I, destiné au traitement du diabète de type I, où le Composé I répond à la formule

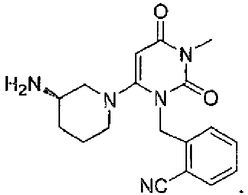

47. Emploi d'un ou de plusieurs composés antidiabétiques différents du Composé I dans la fabrication d'un produit pharmaceutique comprenant une combinaison d'une composition pharmaceutique conforme à l'une quelconque des revendications 1 à 34, 36 et 37 et d'un ou de plusieurs composés antidiabétiques différents du Composé I, destiné au traitement du diabète de type I, où le Composé I répond à la formule
